# EUROPEAN PATENT APPLICATION

(11) **EP 4 183 393 A1**
(43) Date of publication of application: **24.05.2023**
(21) Application number: 21843472.8
(22) Date of filing: 14.07.2021
(51) Int. Cl.: A61K 31/336, A61P 35/00

(54) **COMPOUNDS FOR TREATING HEPATOCELLULAR CARCINOMA**

(30) Priority: 14.07.2020 ES 202030723
(71) Applicant: Servicio Andaluz de Salud, 41071 Sevilla (ES); Universidad De Córdoba, 14004 Córdoba (ES)
(72) Inventor: LOPEZ CANOVAS, Juan Luis, 14004 Córdoba (ES); GAHETE ORTIZ, Manuel D, 14004 Córdoba (ES); LUQUE HUERTAS, Raul M, 14004 Córdoba (ES); DEL RIO MORENO, Mercedes, 14004 Córdoba (ES); CASTAÑO FUENTES, Justo, 14004 Córdoba (ES); RODRÍGUEZ PERÁLVAREZ, Manuel, 14004 Córdoba (ES); DE LA MATA, Manuel, 14004 Córdoba (ES); SÁNCHEZ FRÍAS, Marina Esther, 14004 Córdoba (ES)
(74) Representative: Hoffmann Eitle
(86) International application number: PCT/ES2021/070516
(87) International publication number: WO 2022/013474

(57) **Abstract**

The present invention relates to compounds, compositions, and combined preparations for the treatment of hepatocellular carcinoma, diagnostic methods, and methods for selecting therapeutic agents useful in the prevention, improvement, relief, and/or treatment of hepatocellular carcinoma.

## Description

The present invention is comprised in the field of medicine and relates to compounds for the treatment of hepatocellular carcinoma. Compositions of these compounds and combined preparations, together with other active ingredients, are also provided.

### DESCRIPTION OF THE INVENTION

Liver cancer is a heterogenous pathology that includes various types of cancer of different origins, representing the fourth most common type of cancer worldwide. Hepatocellular carcinoma (HCC) is the most common primary liver tumor (75%) and the most common cause of death in patients with chronic liver disease. It is the sixth most prevalent type of cancer and the second most common cause of cancer-related death due to its high tumor progression and metastasis rate. In fact, HCC often occurs in the context of liver cirrhosis in the vast majority of patients, and any etiology of liver disease, including alcohol consumption, chronic viral hepatitis, and non-alcoholic steatohepatitis, can increase the risk of HCC. Despite the 6-monthly routine liver ultrasound screening strategies, most HCC patients are actually diagnosed in advanced stages in which available or emerging systemic therapies (mainly multikinase inhibitors, antiangiogenics, and cycle checkpoint inhibitors) still have limited impact on overall survival. Therefore, a better understanding of the mechanisms underlying HCC development and progression is considered necessary in order to identify new diagnostic, prognostic, and therapeutic targets.

The treatment, according to the stage of the disease, from the earliest stage to the advanced stage, includes: liver transplant or surgical resection, chemoembolization, or radioembolization, and treatments with angiogenic inhibitors such as sorafenib, regorafenib, or sunitinib. These treatments have a poor efficacy, particularly in more advanced stages, which, along with an increase in incidence, onset of drug resistance, and the lack of diagnostic markers, compels the search for new molecular elements for the treatment of this type of cancer.

### DESCRIPTION OF THE FIGURES

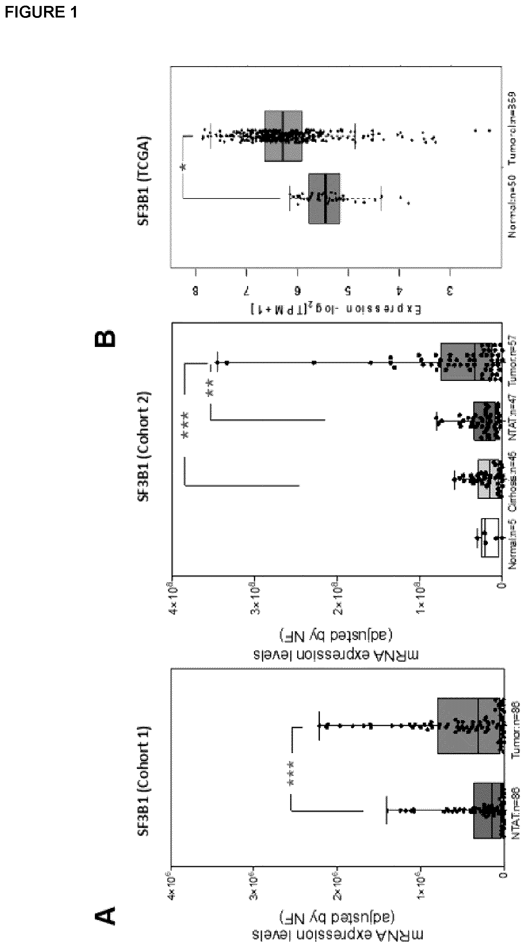

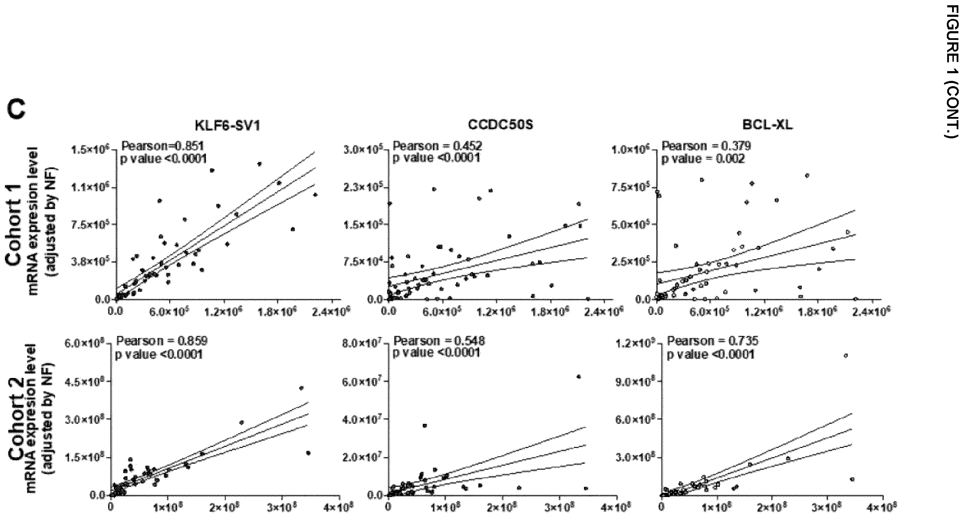

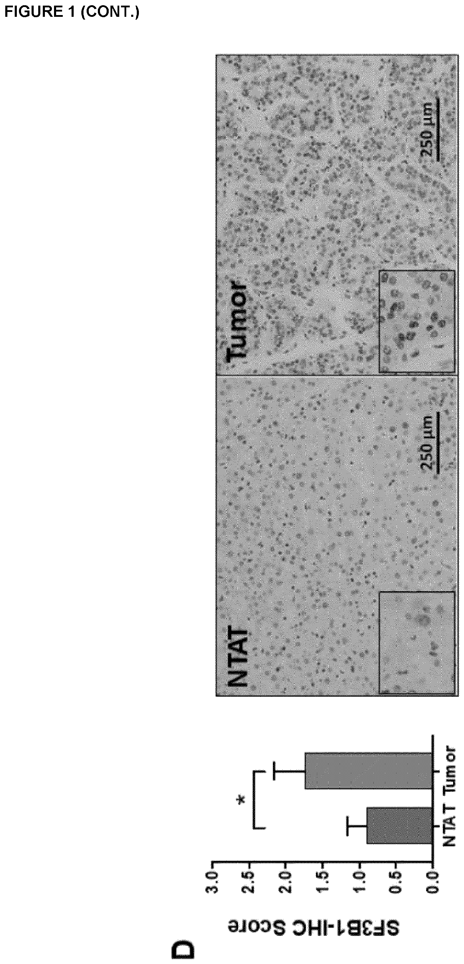

**Figure 1****.** SF3B1 is overexpressed in HCC and correlates with the expression of oncogenic splice variants. (A) SF3B1 expression level in two retrospective cohorts of HCC patients [Cohort-1: FFPE samples (n = 154) and Cohort-2: frozen tissues (n = 172)]. Data is presented as mean ± standard error of the mean. (B) SF3B1 expression level in HCC and normal liver samples from the TCGA cohort. The data represents log2 fold change. (C) Correlations between SF3B1 expression and oncogenic splice variants in HCC samples from both retrospective cohorts. (D) Immunohistochemical assessment of SF3B1 in NTAT and tumor samples (n = 16 patients of Cohort-1). Representative images are depicted (objective X20). Asterisks (* p <0.05; ** p <0.01; *** p <0.001) indicate statistically significant differences. NTAT means adjacent, non-tumor tissue.

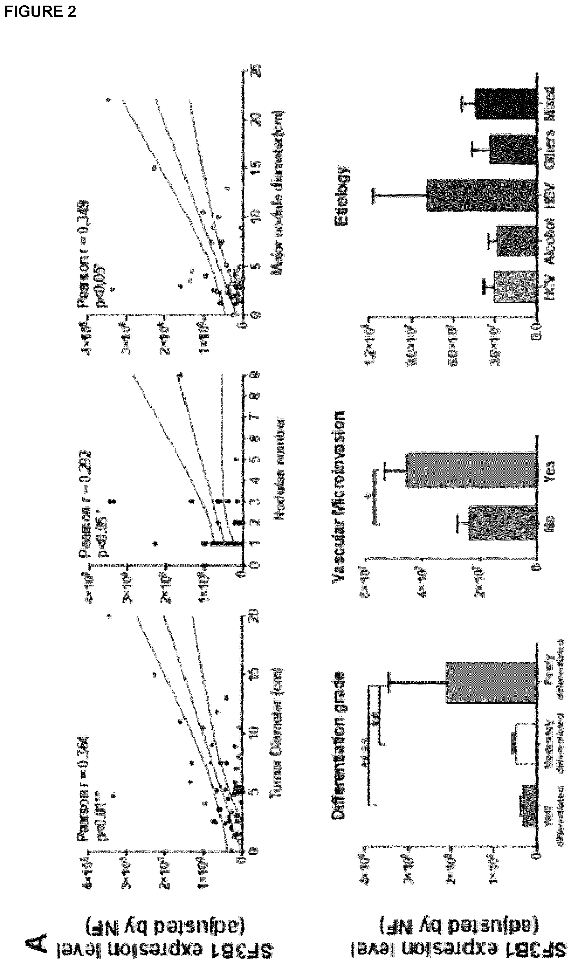

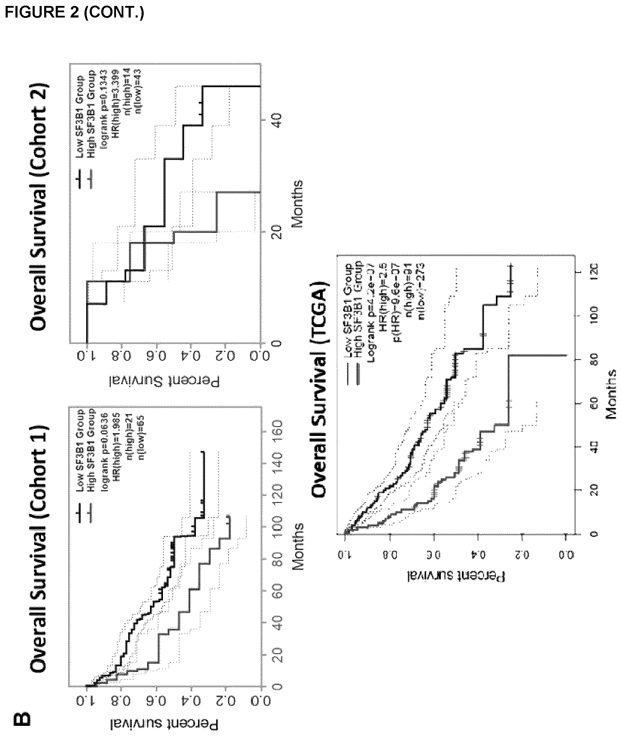

**Figure 2****.** SF3B1 expression is associated with clinical aggressiveness and the low survival of HCC patients. (A) Correlations and associations of SF3B1 expression with aggressiveness parameters in HCC patients of Cohort-2. (B) Overall survival of patients in Cohort-1, Cohort-2, and TCGA categorized by SF3B1 mRNA expression levels (high-expression group = 25% of patients with increased expression compared to the low-expression group = rest of the patients) determined using the long-Rank-p-value method. Asterisks (* p <0.05; ** p <0.01; **** p <0.0001) indicate statistically significant differences. HR means hazard ratio.

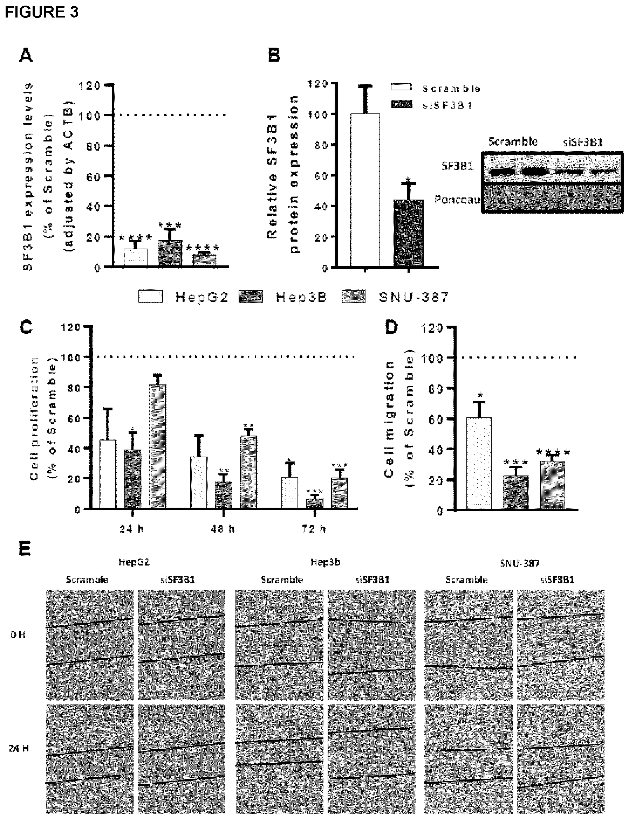

**Figure 3****.** SF3B1 silencing decreases the aggressivity of HCC cell lines. Validation of siRNA-mediated SF3B1 silencing at mRNA (A) and protein (B) levels in HCC cell lines. (C) Proliferation of cells silenced with siSF3B1 in comparison with cells silenced with Scramble. (D) Migration of cells silenced with siSF3B1 in comparison with cells silenced with Scramble. (E) Representative images of cell migration after 24 h. Data is presented as mean ± standard error of the mean of n = 3-5 independent experiments. Asterisks (* p <0.05; ** p <0.01; *** p <0.001; **** p <0.0001) indicate statistically significant differences.

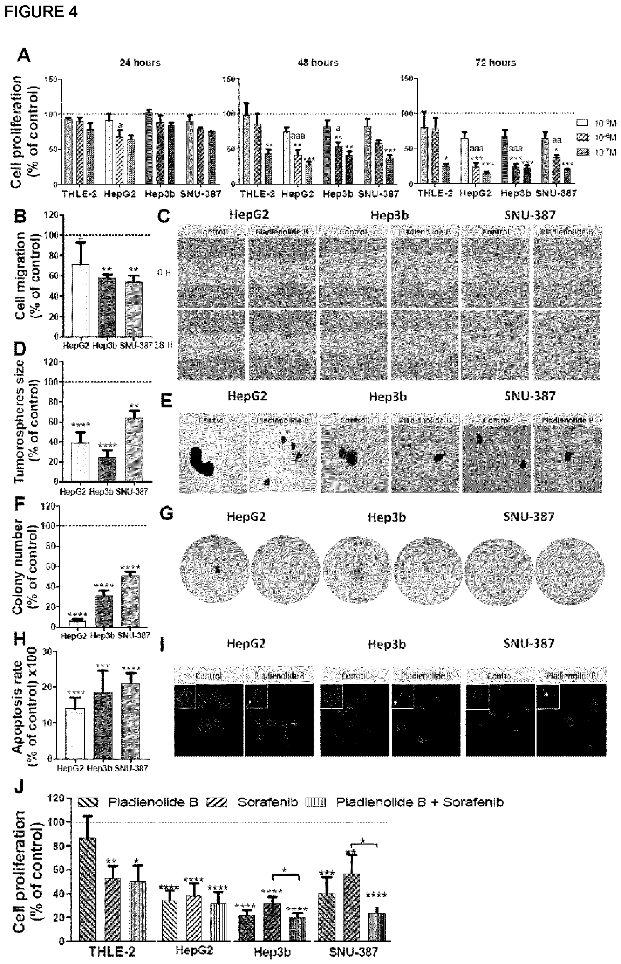

**Figure 4****.** Pharmacological blocking of SF3B1 by pladienolide-B decreases the aggressivity of HCC cell lines. (A) Proliferation of THLE-2, HepG2, Hep3b, and SNU-387 cells treated with pladienolide B (dose-response study) in comparison with cells treated with vehicle. (B) Cell migration of cells treated with pladienolide B (10 nM) in comparison with cells treated with vehicle. (C) Representative images of cell migration after 18h. (D) Mean size of the tumorsphere of cells treated with pladienolide B (10 nM) in comparison with cells treated with vehicle. (E) Representative images of tumorspheres formed after 10 days. (F) Number of colonies formed in cells treated with pladienolide B (10 nM) in comparison with cells treated with vehicle. (G) Representative images of colonies formed after 10 days. (H) Apoptosis rate of cells treated with pladienolide B (10 nM) in comparison with cells treated with vehicle. (I) Representative images of stained nuclei after 24 h. (J) Proliferation rate of THLE-2, HepG2, Hep3b, and SNU-387 cells after 72 h of treatment with pladienolide-B, sorafenib, or the combination thereof in comparison with cells treated with vehicle. Data is presented as mean ± standard error of the mean of n = 3-5 independent experiments. Asterisks (* p <0.05; ** p <0.01; *** p <0.001; **** p <0.0001) indicate statistically significant differences.

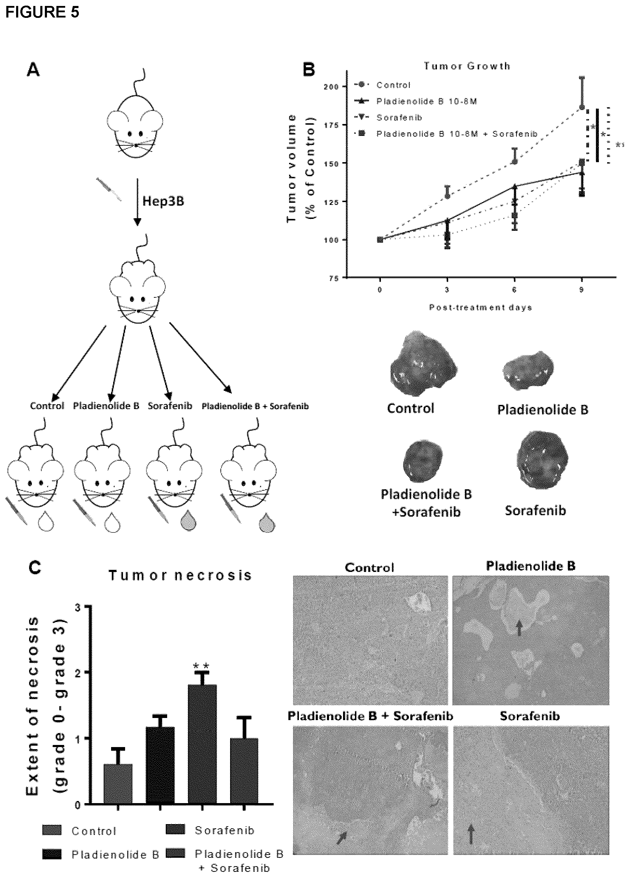

**Figure 5****.** Pharmacological blocking of SF3B1 by pladienolide-B decreases HCC cell growth *in vivo.* (A) Diagram showing the *in vivo* experimental design. In the third week after grafting, the mice were treated with vehicle, pladienolide B, sorafenib, or the combination thereof (n = 4 mice/treatment; n = 8 tumors/treatment. (B) Tumor growth rate was estimated for 9 days after treatment. Representative images of control and treated tumors are depicted. (C) Level of necrosis in tumor xenografts. Representative images of control and treated tumors are depicted. Asterisks (* p <0.05; ** p <0.01) indicate statistically significant differences.

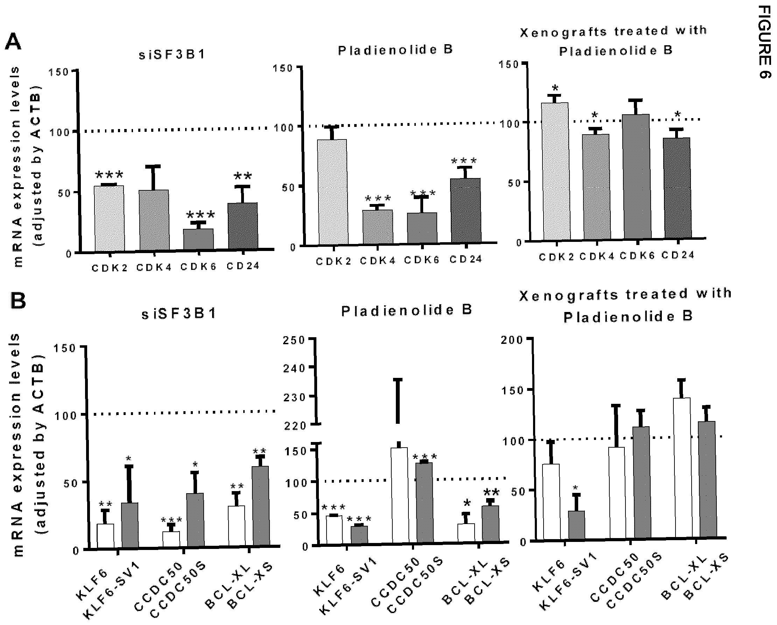

**Figure 6****.** Silencing/blocking of SF3B1 modulates the expression levels of key genes associated with tumors and oncogenic splice variants. Expression levels of key genes associated with tumors (A) or oncogenic splice variants (B) in Hep3b cells treated with siSF3B1 (left panel), Hep3b cells treated with pladienolide B (central panel), and xenotransplanted tumors with Hep3b treated with pladienolide B (right panel) in comparison with the conditions treated with control (codification or vehicle). Data is presented as mean ± standard error of the mean of n = 3-5 independent experiments. Asterisks (* p <0.05; ** p <0.01; *** p <0.001; **** p <0.0001) indicate statistically significant differences.

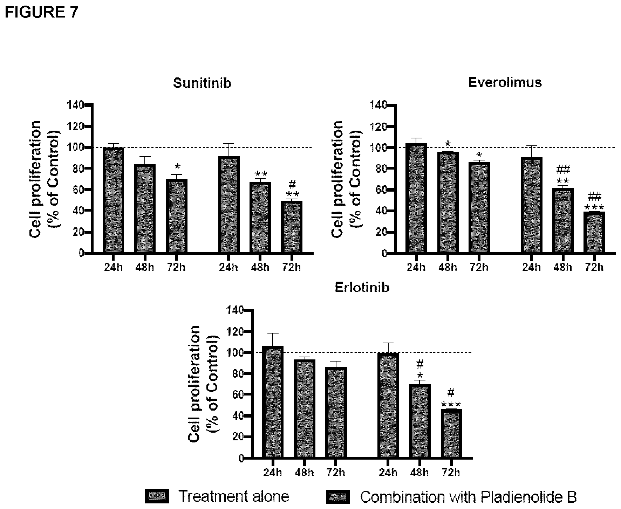

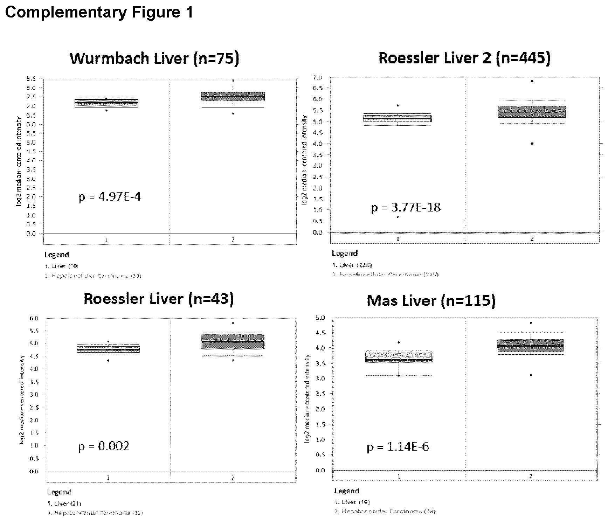

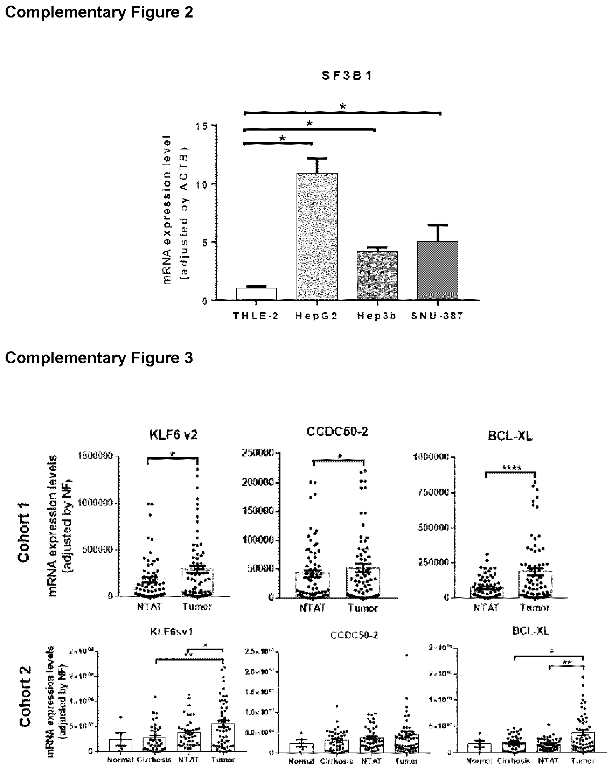

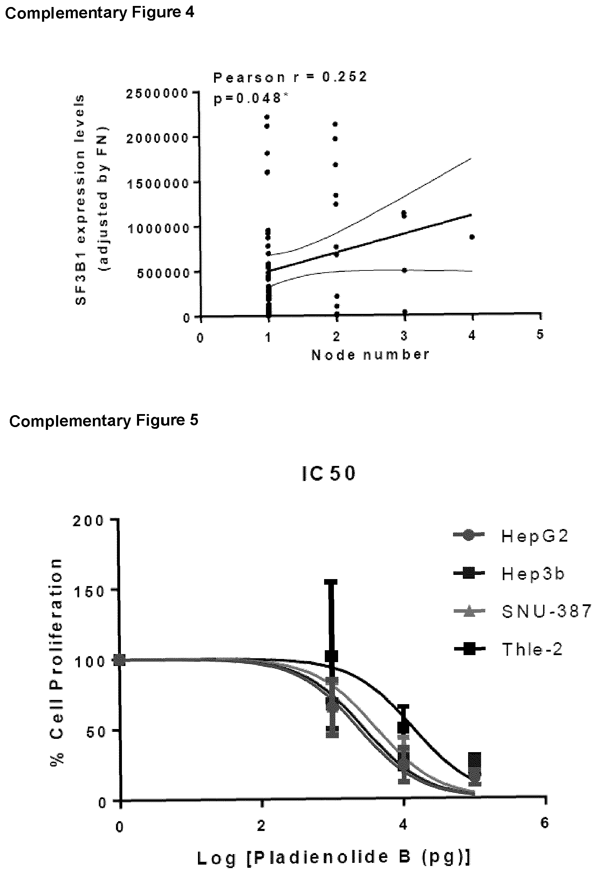

**Figure 7****.** Proliferation rate of Hep3b cells at 24, 48, and 72 h in response to treatment with sunitinib, everolimus, and erlotinib, alone or in combination with pladienolide B, in comparison with cells treated with vehicle (discontinuous line in 100%). Data is presented as mean ± standard error of the mean of n = 3 independent experiments. Asterisks (* p <0.05; ** p <0.01; *** p <0.001) indicate statistically significant differences with respect to control. Hashes (# p <0.05; ## p <0.01) indicate statistically significant differences with respect to individual treatment.

**Table 1. Demographic and clinical parameters of HCC patients.**

| |
|---|
| **Complementary** **Figure 1****.** SF3B1 expression in different cohorts *in silico.* SF3B1 expression in normal tissue in comparison with tumor tissue, the difference of which is significant (p> 0.005). Graphs extracted from the Oncomine database. |
| **Complementary** **Figure 2****.** SF3B1 expression levels in 3 HCC and THLE-2 cell lines. SF3B1 expression in the three HCC lines (n = 4) and in THLE-2 normal liver cell line differed from one another in terms of aggressiveness. Data is expressed in percentages with respect to control (100%). The data shows the mean ± standard error of the mean. |
| Asterisks (* p <0.05; ** p <0.005; *** p <0.001; **** p <0.0001) indicate the existence of statistically significant differences. |
| **Complementary** **Figure 3****.** Expression of the splice variants (KLF1sv1, CCDC50-2, BCL-XL) in cohort 1 and cohort 2. |
| **Complementary** **Figure 4****.** Correlation between SF3B1 expression and node number in cohort 1. Correlation of SF3B1 in the tumor tissue of cohort 1. |
| **Complementary** **Figure 5****.** IC50 assay in response to pladienolide B. Effect of pladienolide B (10-8 M) on the three HCC lines (n = 4) and on THLE-2 normal liver cell line, differed from one another in terms of aggressiveness. 1 nM, 10 nM, 100 nM of pladienolide B was tested 72 hours after treatment. Data is expressed in percentages with respect to control (100%). The data shows the mean ± standard error of the mean. |
| Asterisks (* p <0.05; ** p <0.005; *** p <0.001; **** p <0.0001) indicate the existence of statistically significant differences. |

### DESCRIPTION OF THE INVENTION

The authors of the present invention have studied the possible deregulation and functional pathological implications of SF3B1 in hepatocellular carcinoma (HCC). SF3B1 expression (RNA/protein) and clinical implications were evaluated in HCC patients from two retrospective cohorts (n = 154 and n = 172) and five cohorts *in silico* [TCGA (n = 369), Wurmbach (n = 45), Roessler (n = 43), Roessler 2 (n = 445), and Mas (n = 57)]. The functional and molecular consequences of SF3B1 silencing (siRNA) and/or pharmacological blocking (pladienolide-B) were evaluated in cell lines derived from normal hepatocytes (THLE-2) and liver cancer hepatocytes (HepG2, Hep3b, and SNU-387). Furthermore, Hep3b-induced tumors which were subsequently treated with pladienolide-B were developed *in vivo.*

The inventors observed that SF3B1 expression was consistently elevated (RNA/protein) in HCC compared with control tissues in all the studied cohorts. SF3B1 expression was associated with histological tumor aggressivity, with the expression of oncogenic splice variants (KLF6-SV1, BCL-XL) and a decrease in overall survival. *In vitro* SF3B1 silencing reduced HCC cell line proliferation and migration capacity. Consistently, pladienolide-B, a pharmacological SF3B1 inhibitor, strongly inhibited the proliferation, migration, and formation of colonies and tumors in HCC cells, while having minimal effects on the proliferation of THLE-2. Furthermore, the intratumoral administration of pladienolide-B reduced the *in vivo* growth of xenograft tumors. Lastly, *in vitro*/*in vivo* SF3B1 silencing/blocking significantly modulated the expression of genes associated with cancer (CDK4, CD24) and oncogenic splice variants (KLF6-SV1). The effect of pladienolide-B is comparable to the effect of sorafenib but with a much lower cytotoxicity on normal cells than sorafenib.

Furthermore, as shown in Figure 7, the combined treatment of pladienolide-B together with an antiangiogenic agent improves the effects of the antiangiogenic agent separately. To the knowledge of the inventors, this is the first time hepatocellular carcinoma has been proposed to be treated by combining an antiangiogenic agent and pladienolide-B with advantageous effects.

In summary, SF3B1 is overexpressed in HCC, and its genetic/pharmacological inhibition can represent a new promising therapeutic strategy that is worth exploring through random controlled assays, alone or in combination with existing therapies.

Therefore, a first aspect of the invention relates to an SF3B1 modulating agent, hereinafter modulating agent of the invention, for the prevention, improvement, relief, and/or treatment of hepatocellular carcinoma in an individual.

"Hepatocellular carcinoma", "hepatocarcinoma", "liver cell carcinoma", or "liver cancer" is understood herein to mean cancer which begins in the liver. In most cases, liver cancer is caused by the prolonged damage and healing of the liver (cirrhosis). Cirrhosis can be caused by:
- Excessive alcohol consumption
- Autoimmune diseases of the liver
- Hepatitis B or hepatitis C viral infection
- Prolonged (chronic) liver inflammation
- Iron build-up in the body (hemochromatosis)

People with hepatitis B or C have a high risk of liver cancer, even if they do not have cirrhosis.

SF3B1 (also referred to as splicing factor 3b subunit 1; MDS; PRP10; Hsh155; PRPF10; SAP155; SF3b155) is understood herein to mean both the gene and the protein. This gene encodes subunit 1 of the splicing factor 3b protein complex. Splicing factor 3b, together with splicing factor 3a and a 12S RNA unit, forms the U2 small nuclear ribonucleoprotein complex (U2 snRNP). The splicing factor 3b/3a complex binds pre-mRNA upstream of the intron's branch site in a sequence independent manner and may anchor the U2 snRNP to the pre-mRNA. Splicing factor 3b is also a component of the minor U12-type spliceosome. The carboxy-terminal two-thirds of subunit 1 have 22 non-identical, tandem HEAT repeats that form rod-like, helical structures. Alternative splicing results in multiple transcript variants encoding different isoforms.

In the context of the present invention, SF3B1 is also defined by a nucleotide or polynucleotide sequence, which constitutes the coding sequence of the SF3B1 protein and would comprise several variants originating from:
a) nucleic acid molecules encoding a polypeptide comprising the amino acid sequence of SEQ ID NO: 1,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising an amino acid sequence with an identity of at least 80%, 90%, 95%, 98%, or 99% with SEQ ID NO: 1., wherein the polypeptide encoded by said nucleic acids exhibits the activity and structural characteristics of the SF3B1 protein. Preferably, it is SEQ ID NO: 2

SEQ ID NO: 1 
MAKIAKTHEDIEAQIREIQGKKAALDEAQGVGLDSTGYYDQEIYGGSDSRFAGYVTSIA
ATELEDDDDDYSSSTSLLGQKKPGYHAPVALLNDIPQSTEQYDPFAEHRPPKIADRED
EYKKHRRTMIISPERLDPFADGGKTPDPKMNARTYMDVMREQHLTKEEREIRQQLAE
KAKAGELKVVNGAAASQPPSKRKRRWDQTADQTPGATPKKLSSWDQAETPGHTPSL
RWDETPGRAKGSETPGATPGSKIWDPTPSHTPAGAATPGRGDTPGHATPGHGGAT
 
SSARKNRWDETPKTERDTPGHGSGWAETPRTDRGGDSIGETPTPGASKRKSRWDE
TPASQMGGSTPVLTPGKTPIGTPAMNMATPTPGHIMSMTPEQLQAWRWEREIDERN
RPLSDEELDAMFPEGYKVLPPPAGYVPIRTPARKLTATPTPLGGMTGFHMQTEDRTM
KSVNDQPSGNLPFLKPDDIQYFDKLLVDVDESTLSPEEQKERKIMKLLLKIKNGTPPMR
KAALRQITDKAREFGAGPLFNQILPLLMSPTLEDQERHLLVKVIDRILYKLDDLVRPYVH
KILVVIEPLLIDEDYYARVEGREIISNLAKAAGLATMISTMRPDIDNMDEYVRNTTARAFA
VVASALGIPSLLPFLKAVCKSKKSWQARHTGIKIVQQIAILMGCAILPHLRSLVEIIEHGL
VDEQQKVRTISALAIAALAEAATPYGIESFDSVLKPLWKGIRQHRGKGLAAFLKAIGYLI
PLMDAEYANYYTREVMLILIREFQSPDEEMKKIVLKVVKQCCGTDGVEANYIKTEILPP
FFKHFWQHRMALDRRNYRQLVDTTVELANKVGAAEIISRIVDDLKDEAEQYRKMVME
TIEKIMGNLGAADIDHKLEEQLIDGILYAFQEQTTEDSVMLNGFGTVVNALGKRVKPYL
PQICGTVLWRLNNKSAKVRQQAADLISRTAVVMKTCQEEKLMGHLGVVLYEYLGEEY
PEVLGSILGALKAIVNVIGMHKMTPPIKDLLPRLTPILKNRHEKVQENCIDLVGRIADRG
AEYVSAREWMRICFELLELLKAHKKAIRRATVNTFGYIAKAIGPHDVLATLLNNLKVQE
RQNRVCTTVAIAIVAETCSPFTVLPALMNEYRVPELNVQNGVLKSLSFLFEYIGEMGKD
YIYAVTPLLEDALMDRDLVHRQTASAVVQHMSLGVYGFGCEDSLNHLLNYVWPNVFE
TSPHVIQAVMGALEGLRVAIGPCRMLQYCLQGLFHPARKVRDVYWKIYNSIYIGSQDA
LIAHYPRIYNDDKNTYIRYELDYIL
SEQ ID NO: 2

AGTTCCGTCTGTGTGTTCGAGTGGACAAAATGGCGAAGATCGCCAAGACTCACGA
AGATATTGAAGCACAGATTCGAGAAATTCAAGGCAAGAAGGCAGCTCTTGATGAA
GCTCAAGGAGTGGGCCTCGATTCTACAGGTTATTATGACCAGGAAATTTATGGTG
GAAGTGACAGCAGATTTGCTGGATACGTGACATCAATTGCTGCAACTGAACTTGA
AGATGATGACGATGACTATTCATCATCTACGAGTTTGCTTGGTCAGAAGAAGCCAG
GATATCATGCCCCTGTGGCATTGCTTAATGATATACCACAGTCAACAGAACAGTAT
GATCCATTTGCTGAGCACAGACCTCCAAAGATTGCAGACCGGGAAGATGAATACA
AAAAGCATAGGCGGACCATGATAATTTCCCCAGAGCGTCTTGATCCTTTTGCAGAT
GGAGGGAAAACCCCTGATCCTAAAATGAATGCTAGGACTTACATGGATGTAATGC
GAGAACAACACTTGACTAAAGAAGAACGAGAAATTAGGCAACAGCTAGCAGAAAA
AGCTAAAGCTGGAGAACTAAAAGTCGTCAATGGAGCAGCAGCGTCCCAGCCTCCA
TCAAAACGAAAACGGCGTTGGGATCAAACAGCTGATCAGACTCCTGGTGCCACTC
CCAAAAAACTATCAAGTTGGGATCAGGCAGAGACCCCTGGGCATACTCCTTCCTT
AAGATGGGATGAGACACCAGGTCGTGCAAAGGGAAGCGAGACTCCTGGAGCAAC
CCCAGGCTCAAAAATATGGGATCCTACACCTAGCCACACACCAGCGGGAGCTGCT
ACTCCTGGACGAGGTGATACACCAGGCCATGCGACACCAGGCCATGGAGGCGCA
ACTTCCAGTGCTCGTAAAAACAGATGGGATGAAACCCCCAAAACAGAGAGAGATA

CTCCTGGGCATGGAAGTGGATGGGCTGAGACTCCTCGAACAGATCGAGGTGGAG
ATTCTATTGGTGAAACACCGACTCCTGGAGCCAGTAAAAGAAAATCACGGTGGGA
TGAAACACCAGCTAGTCAGATGGGTGGAAGCACTCCAGTTCTGACCCCTGGAAAG
ACACCAATTGGCACACCAGCCATGAACATGGCTACCCCTACTCCAGGTCACATAA
TGAGTATGACTCCTGAACAGCTTCAGGCTTGGCGGTGGGAAAGAGAAATTGATGA
GAGAAATCGCCCACTTTCTGATGAGGAATTAGATGCTATGTTCCCAGAAGGATATA
AGGTACTTCCTCCTCCAGCTGGTTATGTTCCTATTCGAACTCCAGCTCGAAAGCTG
ACAGCTACTCCAACACCTTTGGGTGGTATGACTGGTTTCCACATGCAAACTGAAG
ATCGAACTATGAAAAGTGTTAATGACCAGCCATCTGGAAATCTTCCATTTTTAAAAC
CTGATGATATTCAATACTTTGATAAACTATTGGTTGATGTTGATGAATCAACACTTA
GTCCAGAAGAGCAAAAAGAGAGAAAAATAATGAAGTTGCTTTTAAAAATTAAGAAT
GGAACACCACCAATGAGAAAGGCTGCATTGCGTCAGATTACTGATAAAGCTCGTG
AATTTGGAGCTGGTCCTTTGTTTAATCAGATTCTTCCTCTGCTGATGTCTCCTACAC
TTGAGGATCAAGAGCGTCATTTACTTGTGAAAGTTATTGATAGGATACTGTACAAA
CTTGATGACTTAGTTCGTCCATATGTGCATAAGATCCTCGTGGTCATTGAACCGCT
ATTGATTGATGAAGATTACTATGCTAGAGTGGAAGGCCGAGAGATCATTTCTAATT
TGGCAAAGGCTGCTGGTCTGGCTACTATGATCTCTACCATGAGACCTGATATAGA
TAACATGGATGAGTATGTCCGTAACACAACAGCTAGAGCTTTTGCTGTTGTAGCCT
CTGCCCTGGGCATTCCTTCTTTATTGCCCTTCTTAAAAGCTGTGTGCAAAAGCAAG
AAGTCCTGGCAAGCGAGACACACTGGTATTAAGATTGTACAACAGATAGCTATTCT
TATGGGCTGTGCCATCTTGCCACATCTTAGAAGTTTAGTTGAAATCATTGAACATG
GTCTTGTGGATGAGCAGCAGAAAGTTCGGACCATCAGTGCTTTGGCCATTGCTGC
CTTGGCTGAAGCAGCAACTCCTTATGGTATCGAATCTTTTGATTCTGTGTTAAAGC
CTTTATGGAAGGGTATCCGCCAACACAGAGGAAAGGGTTTGGCTGCTTTCTTGAA
GGCTATTGGGTATCTTATTCCTCTTATGGATGCAGAATATGCCAACTACTATACTA
GAGAAGTGATGTTAATCCTTATTCGAGAATTCCAGTCTCCTGATGAGGAAATGAAA
AAAATTGTGCTGAAGGTGGTAAAACAGTGTTGTGGGACAGATGGTGTAGAAGCAA
ACTACATTAAAACAGAGATTCTTCCTCCCTTTTTTAAACACTTCTGGCAGCACAGG
ATGGCTTTGGATAGAAGAAATTACCGACAGTTAGTTGATACTACTGTGGAGTTGGC
AAACAAAGTAGGTGCAGCAGAAATTATATCCAGGATTGTGGATGATCTGAAAGATG
AAGCCGAACAGTACAGAAAAATGGTGATGGAGACAATTGAGAAAATTATGGGTAA
TTTGGGAGCAGCAGATATTGATCATAAACTTGAAGAACAACTGATTGATGGTATTC
TTTATGCTTTCCAAGAACAGACTACAGAGGACTCAGTAATGTTGAACGGCTTTGGC
ACAGTGGTTAATGCTCTTGGCAAACGAGTCAAACCATACTTGCCTCAGATCTGTG
GTACAGTTTTGTGGCGTTTAAATAACAAATCTGCTAAAGTTAGGCAACAGGCAGCT
GACTTGATTTCTCGAACTGCTGTTGTCATGAAGACTTGTCAAGAGGAAAAATTGAT
GGGACACTTGGGTGTTGTATTGTATGAGTATTTGGGTGAAGAGTACCCTGAAGTA

TTGGGCAGCATTCTTGGAGCACTGAAGGCCATTGTAAATGTCATAGGTATGCATAA
GATGACTCCACCAATTAAAGATCTGCTGCCTAGACTCACCCCCATCTTAAAGAACA
GACATGAAAAAGTACAAGAGAATTGTATTGATCTTGTTGGTCGTATTGCTGACAGG
GGAGCTGAATATGTATCTGCAAGAGAGTGGATGAGGATTTGCTTTGAGCTTTTAGA
GCTCTTAAAAGCCCACAAAAAGGCTATTCGTAGAGCCACAGTCAACACATTTGGTT
ATATTGCAAAGGCCATTGGCCCTCATGATGTATTGGCTACACTTCTGAACAACCTC
AAAGTTCAAGAAAGGCAGAACAGAGTTTGTACCACTGTAGCAATAGCTATTGTTGC
AGAAACATGTTCACCCTTTACAGTACTCCCTGCCTTAATGAATGAATACAGAGTTC
CTGAACTGAATGTTCAAAATGGAGTGTTAAAATCGCTTTCCTTCTTGTTTGAATATA
TTGGTGAAATGGGAAAAGACTACATTTATGCCGTAACACCGTTACTTGAAGATGCT
TTAATGGATAGAGACCTTGTACACAGACAGACGGCTAGTGCAGTGGTACAGCACA
TGTCACTTGGGGTTTATGGATTTGGTTGTGAAGATTCGCTGAATCACTTGTTGAAC
TATGTATGGCCCAATGTATTTGAGACATCTCCTCATGTAATTCAGGCAGTTATGGG
AGCCCTAGAGGGCCTGAGAGTTGCTATTGGACCATGTAGAATGTTGCAATATTGT
TTACAGGGTCTGTTTCACCCAGCCCGGAAAGTCAGAGATGTATATTGGAAAATTTA
CAACTCCATCTACATTGGTTCCCAGGACGCTCTCATAGCACATTACCCAAGAATCT
ACAACGATGATAAGAACACCTATATTCGTTATGAACTTGACTATATCTTATAATTTT
ATTGTTTATTTTGTGTTTAATGCACAGCTACTTCACACCTTAAACTTGCTTTGATTTG
GTGATGTAAACTTTTAAACATTGCAGATCAGTGTAGAACTGGTCATAGAGGAAGAG
CTAGAAATCCAGTAGCATGATTTTTAAATAACCTGTCTTTGTTTTTGATGTTAAACA
GTAAATGCCAGTAGTGACCAAGAACACAGTGATTATATACACTATACTGGAGGGAT
TTCATTTTTAATTCATCTTTATGAAGATTTAGAACTCATTCCTTGTGTTTAAAGGGAA
TGTTTAATTGAGAAATAAACATTTGTGTACAAAATGCTAATTTGTGTGTGTTTTTTGA
ACATGACTTGTAAAATGCGGAACTTTGATAAAGTATTGGTTTATGTGGAATAAGTG
GCTTAATTTCATTTTCTGTCACATGGTTTATAGAAAGTAGTTAGCTGAATAAAAACT
ATATAAAGTGATGGCATCTTTGTCAAAATTCCATTGTTGTGTTAATAATGACAGCAA
GAAGAGTAGCCTCAGGGGATGTTCCCCTCAAACTAGCACAACCATTCCATCATCG
TAGAAAAGTAGCACTTTTGCTAAACTGTCTTGAATATTTTGTACTTACATAGCGCCT
TTCATCTCTTGATTTCTCAAAATGCTTTATGAACACATTTAAAGAAAGTGGTTTAAG
TCTTGTCCAACACTTGACAGGTCTGCTGTGTTTAGCAAGTGAGGAATTTAACTTTA
CTTCAAAACTGCTTTCTGCCTATTAGGAGTGAGGATACCTAAGTAATGCTGATAGA
ACAGGACAATGTTGGGCTTTTCTCCATGTTATAAGCCACTACTCAGCAATGCATCA
GTAAATACCTATTCACCCACTGTATGCCAGCCACTGTGCTTATATGCAGGGGATG
CAAAGGTGCATGAGACCTGCCCTCTACCTTTAAGAACAGTATAATGGAGAAGGGG
AGACAAACCTTGTGACAGATTCCTTATGTACTGTGATAATACACAGTGGCAGCAGC
TAAAATGTCTAGGTTTGCTTAGCTTTGTATTCAGTAATAATAAGTTGATCTAAGAGT
TCAGCATAAACTGAATGAAATGCCATTTAATGGTAGAGGAACCAAGCATTAAGGCA

GTACTACACTTAATTTTTTAAGCAAATATGTAAAGTATATTTTCAAACTTTTCTAATG
TTATGGCCCCAAATTTCTTAGTTTTGGCCTTTTTACTACCTATACTATTTTTACTGTT
TTGTTTTGTCTCCATGTGGTAGTACTTCTGTGAACTCTAAAGGGAAAAAAAATTCTG
CAAGCAGCATTAGTATATAACTACTACTGTAAGTAAAACTGCCTATTGACACTTTAG
GAGTTCCTGCTTCAGAAGCTTAGTTAAGAAACAGCTTGTGGCCGGGTGTGGTGGC
TCATGCCTGTAATCCCAGCACTTTGGGAGGCTGAGGCGGGCGGATCACGAGGTC
AGGAGATCGAGACCATCCTGGCTAACGCGGTGAAGTCCCGTCTACTAAAAATACA
AAAATTAGCCAGGCGTGGTGGCGGGCGCCTGTAGTCCCAGCTACTAGGGAGGCT
GAGGCAGGAGAATGGCTTGAACCCGGGAGGCGGAGCTTGCAGTGAGCCGAGAT
AGCGCTGCTACACTCCAGCCTGGGCGACAGAGACTCCGTCTCAAAAAAAAAAAAA
AAATTGACTTTCAACAAATTGATAGTGAGCATTAAGGGTTTCCAAGTTGGATTTGTA
ACTCCTCATCATTCCTTGTATGACAACTTTCTGAATATATGTCACTATGTAGTAAAA
TTAAACACTCCAAACTCATCTTTCTGTTGTTAGAAGTTTTCAGCGGTACTTCCATGC
AACTTTAAATCTCACTGCTCTCTATGGTTGATGTCAAATGACCTTCAGTAATGACTG
AGAATTGAATACAAATAGATTACAAAGCCAAAATTTGATGTTAAATGACTCAGGAAA
TTTTAGTTGTATTTTCAATTCAAGTACTTAGTAGCCTACGTTTGCTTGGCCTCTGGT
TCTTTATGGAAAATAGGCTTTGTAGTGGCATTGTGGAGCAAAGGAGACTGTTACAC
CTTAATTAACTTTTTTTACTGATGCAAATAATTTGAGGATAGAGAGGAGGGAAGTA
GTGAAAGCTATGACCTAAAACATTGGGACCAAATAGAGGCTCACAGATATTTGGAT
TATTTTATGTGCTTATTATTAAATAAGGAAAGCATTTTGTGATATGTGGAAGACGCT
ATGTGAAGTTTTACCTATCTTCTCAAAGACCTTTTCTTTTGTATTTTCTTTTGGTGTT
TCTTAAAGCCAAACAAAGAAATGTTCTTAAGGAGACAGGGTGGGTTTTTCTGTGGG
CCTTTGTTGGTTTTTCTGTGGGCCATCGCCCTCTAATGGAATTGATCTCTGGCTGT
TTGATTTTTTTCATATTGTATTTTTAAAATTTGTTGTACAGTGCCCTGTGAGCACCAA
GTACCACTAGATGAATAAAACGTATTATATCTAAA

In a preferred embodiment of this aspect of the invention, the individual has hepatocellular carcinoma that is resistant to an antiangiogenic agent.

"Antiangiogenic agent" is understood herein to mean a chemical or biological agent which inhibits or reduces the formation of new blood vessels from pre-existing vessels (angiogenesis). There are many natural angiogenesis inhibitors which help to maintain control over the formation of blood vessels, such as angiostatin, endostatin, and thrombospondin, among others.

These agents are often used to combat cancer by destroying immature blood vessels recently formed in the tumor, depleting tumor cells of nutrients and oxygen and thereby inhibiting tumor growth.

Preferably, the antiangiogenic agent is selected from: axitinib (Inlyta^{®}), Bevacizumab (Avastin^{®}), cabozantinib (Cometriq^{®}), everolimus (Afinitor^{®}), lenalidomide (Revlimid^{®}), lenvatinib mesylate (Lenvima^{®}), pazopanib (Votrient^{®}), ramucirumab (Cyramza^{®}), regorafenib (Stivarga^{®}), sorafenib (Nexavar^{®}), sunitinib (Sutent^{®}), thalidomide (Synovir, Thalomid^{®}), vandetanib (Caprelsa^{®}), ziv-aflibercept (Zaltrap^{®}).

Even more preferably, the antiangiogenic agent is the compound sorafenib.

SF3B1 activity can be modulated by modifying SF3B1 protein levels and/or activity, or by modifying SF3B1 gene transcription levels such that SF3B1 protein activity levels in the cell is modulated. In the context of the present invention, inhibition is the preferred way of modulation.

SF3B1 antagonists are known in the state of the art.

Therefore, in a preferred embodiment of this aspect of the invention, the modulating agents comprised in the composition of the invention are selected from a list comprising:
a) an organic molecule,
b) an RNA molecule,
c) an antisense oligonucleotide,
d) an antibody, or e) a ribozyme.

Nucleotide sequences specifically complementary to a specific DNA or RNA sequence may form complexes and block transcription or translation. In that sense, with the progress of post-transcriptional gene silencing, and particularly of RNA interference (RNAi), tools which allow the specific inhibition of gene expression have been developed. The inhibition of SF3B1 protein expression would therefore constitute the inhibition of its biological activity, and specifically, would be useful in the treatment of hepatocellular carcinoma.

"Antisense polynucleotides" are understood to mean ribonucleotide or deoxyribonucleotide strands which can inhibit SF3B1 production by one of these three mechanisms:
1. Interfering with transcription by hybridizing in the structural gene or in a regulatory region of the gene encoding SF3B1. Since the hybridization of the antisense oligonucleotide with the DNA effectively blocks transcription or expression, it reduces SF3B1 production.
2. Binding of the antisense oligonucleotide with mRNA in the cytoplasm, interfering with the formation of the translation construct *per se,* inhibiting mRNA translation into protein.
3. Forming an mRNA-antisense duplex which allows a rapid degradation of the duplex mRNA by RNases (such as RNase H). This results in a lower SF3B1 production.

Antisense oligonucleotides capable of inhibiting SF3B1 are known in the state of the art. For example, and without limitation, it could be a ribonucleotide or RNA sequence from the so-called siRNA (small interfering RNA) or silencing RNA, capable of inhibiting SF3B1 gene expression. In the context of the present specification, "siRNA" (small interfering RNA) is understood to mean a class of double-stranded RNA having a length of 19 to 25 nucleotides, and more preferably between 21 and 23 nucleotides, that is involved in the RNA interference pathway, where the siRNA interferes with the expression of a specific gene. In the present invention, this specific gene is SF3B1.

These oligonucleotides could also be an RNA construct which contains at least any one of the possible siRNA nucleotide sequences capable of inhibiting SF3B1 expression, and notwithstanding the fact that, any of the RNA sequences and constructs of the invention described above which are object of preferably chemical modifications that would lead to a greater stability against the action of ribonucleases and thereby a greater efficiency, are also part of the present invention, without said modifications entailing the alteration of their mechanism of action, i.e., specific binding to RISC complex (RNA-induced silencing complex), activating it and giving rise to helicase activity which separates the two strands, leaving only the antisense strand associated with the complex. The resulting ribonucleoprotein complex binds to the target mRNA (SF3B1 messenger RNA, listed in SEQ ID NO: 2). If the complementarity is not perfect, RISC associates with the messenger and translation is attenuated. However, if complementary is perfect, RISC will act as an RNase, cutting the messenger and being free to repeat the process.

Additionally, it is evident for one skilled in the art that a large amount of mRNA polynucleotides can be translated into SF3B1 as a result, for example, of the genetic code being degenerated. Any siRNA capable of inhibiting the translation of these mRNA is also part of the invention.

The preparation of the siRNA sequence of the invention or of the RNA construct of the invention would be evident for one skilled in the art, and could be carried out by chemical synthesis, which furthermore allows both the incorporation of chemical modifications in the different nucleotides of the product and the incorporation of other chemical compounds at any of the ends. Moreover, synthesis could also be performed enzymatically using any of the RNA polymerases available. Enzymatic synthesis also allows chemical modification of the RNA products or inhibitors.

The design of the siRNA nucleotide sequence of the invention would also be evident for one skilled in the art. In that sense, it could be performed by means of a random design in which 19-25 bases are selected from the target mRNA without taking into account the sequence or positional information of the transcript. Another non-limiting alternative of the present invention would be conventional design by means of simple parameters developed by the pioneers of the technique (Calipel, A. et al., 2003. J Biol Chem. 278(43): 42409-42418) completed with a BLAST nucleotide analysis. Another possibility could be a rational design in which a computerized method aimed at identifying optimal siRNA targets in an mRNA is used. The target sequences are analyzed in groups of 19 nucleotides at the same time and the sequences having the best characteristics are identified based on an algorithm which incorporates a large number of thermodynamic and sequence parameters.

A DNA gene construct could also be part of the composition of the invention, which construct would direct the *in vitro* or intracellular transcription of the siRNA sequence or RNA construct of the invention and comprises at least one of the following types of sequences: a) a preferably double-stranded DNA nucleotide sequence comprising at least the coding sequence of the siRNA of the invention or of the RNA construct of the invention for transcription thereof, or b) a preferably double-stranded DNA nucleotide sequence corresponding to a gene expression system or vector comprising the coding sequence of the RNA sequence of the invention operatively linked with at least one promotor that directs the transcription of said nucleotide sequence of interest, and with other sequences required or suitable for transcription and appropriate regulation in time and space, for example, start and end signals, cleavage sites, polyadenylation signal, origin of replication, transcriptional enhancers, transcriptional silencers, etc., for use in those pathological contexts in which SF3B1 is contributing to the worsening of sympathetic overactivation. Several of these expression constructs, systems, or vectors can be obtained by means of conventional methods known by those skilled in the art (Sambrook et al. 2001. Molecular Cloning: A Laboratory Manual. Cold Spring Harbor Laboratory Press, New York)

The compositions of the present invention allow the *in vivo* or *in vitro* transfection of the siRNA of the invention into a cell. Transfection could be carried out, but without limitation to, direct transfection or vectors which facilitate the access of the siRNA into the cell. In that sense, examples of these vectors are, without limitation, retrovirus, lentivirus, adenovirus, adeno-associated virus, *Herpes simplex* virus, non-viral DNA plasmids, cationic liposomes, and molecular conjugates. In that sense, for example, the siRNAs of the present invention, as well as the precursor RNAs or DNAs of these siRNAs, can be conjugated with release peptides or other compounds to favor the transport of these siRNAs into the cell.

As it is used herein, the term "antibody" refers to immunoglobulin molecules and immunologically active portions of immunoglobulin molecules, i.e., molecules that contain an antigen-binding site which binds specifically (reacts immunologically with) the SF3B1 protein. Examples of immunologically active portions of immunoglobulin molecules include F(ab) and F(ab')2 fragments which can be generated by treating the antibody with an enzyme such as pepsin. It can be a monoclonal or polyclonal antibody.

The antibodies capable of binding to the SF3B1 protein can be used to inhibit the activity of said protein. Such antibodies are commercially available and/or can be readily obtained by one skilled in the art by means of known methods. The antibodies or fragments thereof may be capable of inhibiting the activity of the SF3B1 protein that contributes to hepatocellular carcinoma. The antibodies can be polyclonal (typically include different antibodies directed against different determinants or epitopes) or monoclonal (directed against a single determinant in the antigen). Monoclonal antibody can be biochemically altered by genetic manipulation, or can be synthetic with the antibody possibly lacking, as a whole or in parts, portions which are not required for SF3B1 recognition and which are replaced by other portions that give additional advantageous properties to the antibody. The antibody can be also recombinant, chimeric, humanized, synthetic, or a combination of any of the foregoing.

A "recombinant antibody or polypeptide" (rAb) is an antibody or polypeptide produced in a host cell transformed or transfected with the nucleic acid coding the polypeptide or producing the polypeptide as a result of homologous recombination.

These rAbs can be expressed in and directed to specific cellular subcompartments when suitable sequences for intracellular traffic are incorporated therein. These antibodies are referred to as intrabodies, and their efficacy has been proven not only in diverting proteins from its usual compartment or blocking interactions between proteins involved in signaling pathways, but also in activating intracellular proteins.

DNA gene constructs capable of being transcribed into a peptide, antibody, or antibody fragment, for use against SF3B1, and in the treatment of pathologies occurring with sympathetic overactivation are also part of the invention. Said DNA gene construct would direct *in vitro* or intracellular transcription of the sequence of the antibody or fragment thereof, and comprises at least one of the following types of sequences: a) a preferably double-stranded DNA nucleotide sequence comprising at least the coding sequence of the antibody of the invention or of the antibody fragment of the invention for *in vitro* or intracellular transcription, b) a preferably double-stranded DNA nucleotide sequence corresponding to a gene expression system or vector comprising the coding sequence of the sequence of the antibody or antibody fragment of the invention operatively linked with at least one promotor that directs the transcription of said nucleotide sequence of interest, and with other sequences required or suitable for transcription and appropriate regulation in time and space, for example, start and end signals, cleavage sites, polyadenylation signal, origin of replication, transcriptional enhancers, transcriptional silencers, etc., for use in those pathological contexts occurring with an infection.

As it is understood herein, a "ribozyme" refers to a catalytic polynucleotide (typically RNA) which can be constructed to specifically recognize an mRNA by hybridization and to fragment it or eliminate its expression. Ribozymes can be introduced into the cell as catalytic RNA molecules or as gene constructs which express catalytic RNA molecules.

One skilled in the art would be able to prepare organic molecules that can bind specifically to SF3B1 without binding to other polypeptides or proteins. Organic molecules will preferably have a weight of 100 to 20,000 Daltons, more preferably 500 to 15,000 Daltons, and more preferably 1000 to 10,000 Daltons. Organic molecule libraries are commercially available. The administration route can be, without limitation, intraperitoneal, intrathecal, intravenous, intramuscular, subcutaneous, intraventricular, oral, enteral, parenteral, intranasal, or dermal.

Therefore, in another preferred embodiment of this aspect of the invention, the SF3B1 modulating agent is an inhibitor, and more preferably a compound of formula (I), hereinafter the compound of the invention: 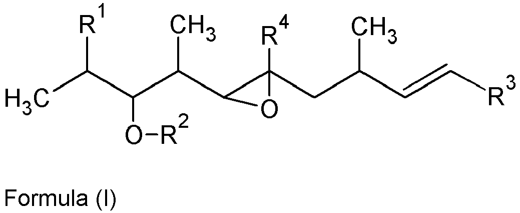 or a pharmaceutically acceptable salt thereof, wherein:
R¹ is H, OH, or alkoxy;
R² is H or CH3;
R⁴ is H or CH3;
R³ is alkyl, cycloalkyl, aryl, or heterocycloalkyl;

In other words, one aspect of the invention relates to a compound of formula (I) or a pharmaceutically acceptable salt thereof, for the prevention, improvement, relief, or treatment of hepatocellular carcinoma in an individual.

The compounds described herein can inhibit an enzyme or a biochemical pathway. The terms "inhibit" and "inhibition" refer to slowing down, stopping, or reverting the growth or progression of a disease, infection, condition, route, or group of cells. Inhibition can be greater than about 20%, 40%, 60%, 80%, 90%, 95%, or 99%, for example, in comparison with the growth or progression occurring in the absence of treatment or contact.

The term "alkyl" refers to a straight chain, branched chain, and/or cyclic ("cycloalkyl") saturated hydrocarbon having 1 to 20 (for example, 1 to 10 or 1 to 4) carbon atoms. Alkyl moieties having 1 to 4 carbons are referred to as "lower alkyl". Examples of alkyl groups include, but are not limited to, methyl, ethyl, propyl, isopropyl, n-butyl, t-butyl, isobutyl, pentyl, hexyl, iso hexyl, heptyl, 4,4-dimethylpentyl, octyl, 2,2,4-trimethylpentyl, nonyl, decyl, undecyl, and dodecyl. The term "alkyl" can include "alkenyl" and "alkynyl" moieties. An alkyl can be substituted with a cycloalkyl. An example of a cycloalkyl group substituted with an alkyl is 1-ethyl-4-methyl-cyclohexyl, and the group can bind to a compound in any carbon atom of the group.

The term "cycloalkyl" refers to a cyclic hydrocarbon having 1 to 20 (for example, 1 to 10 or 1 to 6) carbon atoms. Examples of cycloalkyl groups include, without limitation, cyclopropyl, cyclobutyl, cyclopentyl, cyclohexyl, and adamantyl.

The term "alkenyl" refers to a straight chain, branched chain, and/or cyclic hydrocarbon having 2 to 20 (for example, 2 to 10 or 2 to 6) carbon atoms, and includes at least one carbon-carbon double bond. Representative alkenyl moieties include vinyl, allyl, 1-butenyl, 2-butenyl, isobutylene, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, and 3-decenyl.

The term "alkoxy" refers to an -O-alkyl group. Examples of alkoxy groups include, but are not limited to, -OCH₃, -OCH₂CH₃, -O(CH₂)₂CH₃, -O(CH₂)₃CH₃, -O(CH₂)₄CH₃, and -O(CH₂)₅CH₃.

The term "aryl" refers to an aromatic ring or a system of aromatic or partially aromatic ring made up of carbon and hydrogen atoms. An aryl moiety can comprise multiple rings attached or fused to one another. Examples of aryl moieties include, but are not limited to, anthracenyl, azulenyl, biphenyl, fluorenyl, indan, indenyl, naphthyl, phenanthrenyl, phenyl, 1,2,3,4-tetrahydro-naphthalene, and tolyl.

The term "heteroalkyl" refers to an alkyl moiety in which at least one of its carbon atoms has been replaced by a heteroatom (for example, N, O, or S).

The term "heteroaryl" refers to an aryl moiety in which at least one of its carbon atoms has been replaced by a heteroatom (for example, N, O, or S). Examples include, but are not limited to, acrydinyl, benzimidazolyl, benzofuranyl, benzoisothiazolyl, benzoisoxazolyl, benzoquinazolynyl, benzothiazolyl, benzoxazolyl, furyl, imidazolyl, indolyl, isothiazolyl, pyrilazol, pyrilazyl, pyrazinyl, pyramido, pyrazyl, pyrimidyl, pyrrolyl, quinazolynyl, quinolinyl, tetrazolyl, thiazolyl, and triazinyl.

The term "heteroarylalkyl" refers to a heteroaryl moiety attached to an alkyl moiety.

The term "heterocycle" refers to a monocyclic or polycyclic ring or a monocyclic or polycyclic aromatic, partially aromatic, or non-aromatic ring system made up of carbon, hydrogen, and at least one heteroatom (for example, N, O, or S). A heterocycle can comprise multiple (i.e., two or more) rings fused or attached to one another. Heterocycles can include heteroaryls. Examples include, but are not limited to, benzo[1,3]dioxolyl, 2,3-dihydro-benzo[1,4]dioxynyl, cynolinyl, furanyl, hydantoinyl, morpholinyl, oxetanyl, oxyranyl, piperazinyl, piperidinyl, pyrrolidonyl, pyrrolidinyl. tetrahydrofuranyl, tetrahydropyranyl, tetrahydropyridinyl, tetrahydropyrimidinyl, tetrahydrothiophenyl, tetrahydrothiopyranyl, and valerolactamyl.

The term "heterocycloalkyl" refers to a non-aromatic heterocycle.

The term "substituted", when used to describe a chemical moiety or structure, refers to a derivative of said structure or moiety in which one or more its hydrogen atoms is substituted with a chemical moiety or functional group such as, but not limited to, alcohol (for example, hydroxyl, alkyl-OH), aldehyde, alkanoyloxy, alkoxycarbonyl, alkyl (for example, methyl, ethyl, propyl, t-butyl), alkenyl, alkynyl, alkylcarbonyloxy (-OC(O)R), amide (-C(O)NHR- or -RNHC(O)-), amidinyl (-C(NH)NHR or -C(NR)NH₂), amine (primary, secondary, and tertiary such as alkylamino, arylamino, arylalkylamino), aroyl, aryl, aryloxy, azo, carbamoyl (-NHC(O)OR- or -OC(O)NHR-), carbamyl (for example, - CONH₂, as well as CONH-alkyl, CONH-aryl and CONH-arylalkyl (for example, Bn)), carbonyl, carboxyl, carboxylic acid, carboxylic acid anhydride, carboxylic acid chloride, cyano, ester, epoxide, ether (for example, methoxy, ethoxy), guanidine, imine (primary and secondary), isocyanate, isothiocyanate, ketone, halo (F, Cl, Br, or I), haloalkyl (for example, fluoromethyl, difluoromethyl, trifluoromethyl), hemiacetal, heterocycle, nitrile, nitro, phosphodiester, sulfur, sulfonamido (for example, SO₂NH₂), sulfone, sulfonyl (included including alkylsulfonyl, arylsulfonyl, and arylalkylsulfonyl), sulfoxide, thiol (for example, sulfhydryl, thioether) and urea (-NHCONHR-). The aforementioned groups can be elements of a group R as described herein, including R, R1, R², R³, R⁴, and/or R⁵. Furthermore, one or more of the aforementioned groups can be explicitly excluded from a definition of one of the aforementioned groups R.

The term "pharmaceutically acceptable salts" refers to salts prepared from non-toxic pharmaceutically acceptable acids or bases including inorganic acids and bases and organic acids and bases. Suitable pharmaceutically acceptable base addition salts include, among others, metal salts of aluminum, calcium, lithium, magnesium, potassium, sodium, and zinc or organic salts of lysine, N,N-dibenzylethylenediamine, chloroprocaine, choline, diethanolamine, ethylenediamine, meglumine (N-methylglucamine), and procaine. Suitable non-toxic acids include, among others, inorganic and organic acids such as acetic, alginic, anthranilic, benzenesulfonic, benzoic, camphorsulfonic, citric, ethanesulfonic, formic, fumaric, furoic, galacturonic, gluconic, glucuronic, glutamic, glycolic. hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric, and p-toluenesulfonic acids. Specific non-toxic acids include hydrochloric, hydrobromic, phosphoric, sulfuric, and methanesulfonic acids. Examples of specific salts therefore include hydrochloride and mesylate salts. Other acids are well known in the art. See, for example, Remington's Pharmaceutical Sciences (18a ed., Mack Publishing, Easton Pa.: 1990) and Remington: The Science and Practice of Pharmacy (19th ed., Mack Publishing, Easton Pa.: 1995).

In a more preferred embodiment of this aspect of the invention, the compound of formula (I) is pladienolide B of formula (II):

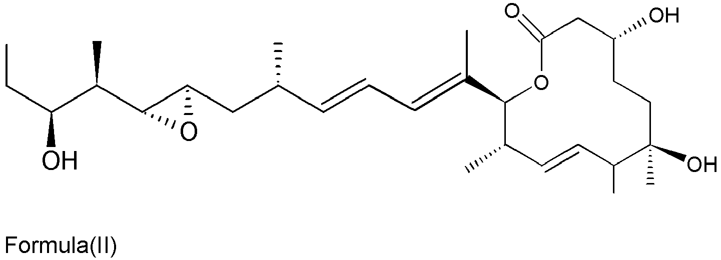

Pladienolide B, or [(2S,3S,4E,6S,7R,10R)-7,10-dihydroxy-2-[(2E,4E,6S)-7-[(2R,3R)-3-[(2R,3S)-3-hyd roxypentan-2-yl]oxyran-2-yl]-6-m ethyl hepta-2,4-dien-2-yl]-3,7-dimethyl-12-oxo-1-oxacyclododec-4-en-6-yl]acetate, is a compound with CAS number: 445493-23-2.

### PHARMACEUTICAL COMPOSITION AND DOSAGE FORM OF THE INVENTION

Another **aspect** of the invention relates to a composition comprising the compound of the invention for the prevention, improvement, relief, or treatment of hepatocellular carcinoma in an individual. Preferably, the composition of the invention is a pharmaceutical composition. More preferably, it further comprises a pharmaceutically acceptable vehicle. Even more preferably, the composition of the invention further comprises another active ingredient.

In another preferred embodiment of this aspect of the invention, the individual has hepatocellular carcinoma that is resistant to an antiangiogenic agent.

Preferably, the antiangiogenic agent is selected from: axitinib (Inlyta^{®}), bevacizumab (Avastin^{®}), cabozantinib (Cometriq^{®}), everolimus (Afinitor^{®}), lenalidomide (Revlimid^{®}), lenvatinib mesylate (Lenvima^{®}), pazopanib (Votrient^{®}), ramucirumab (Cyramza^{®}), regorafenib (Stivarga^{®}), sorafenib (Nexavar^{®}), sunitinib (Sutent^{®}), thalidomide (Synovir, Thalomid^{®}), vandetanib (Caprelsa^{®}), ziv-aflibercept (Zaltrap^{®}).

Even more preferably, the antiangiogenic agent is the compound sorafenib.

Preferably, the other active ingredient is an antiangiogenic agent for the prevention, improvement, relief, or treatment of hepatocellular carcinoma.

The pharmaceutically acceptable adjuvants and vehicles which can be used in said compositions are adjuvants and vehicles known by those skilled in the art and commonly used in the preparation of therapeutic compositions.

In the sense used herein, the expression "therapeutically effective amount" refers to the amount of the agent or compound capable of developing specific therapeutic action as a result of its pharmacological properties, calculated to produce the desired effect and will generally be determined, among other factors, by the characteristics typical of the compounds, including age, patient condition, the severity of the alteration or illness, and the administration route and frequency.

The compounds described in the present invention, their salts, prodrugs, and/or solvates, as well as the pharmaceutical compositions containing same can be used together with other additional drugs or active ingredients so as to provide a combination therapy. Said additional drugs can be part of the same pharmaceutical composition or, alternatively, can be provided in the form of a separate composition for simultaneous or non-simultaneous administration along with the pharmaceutical composition of the invention.

Another **aspect** of the invention relates to a dosage form, hereinafter dosage form of the invention, which comprises a compound of the invention or the composition of the invention.

"Dosage form" is understood herein to mean the mixture of one or more active ingredients with or without additives having physical characteristics for suitable dosing, storage, administration, and bioavailability.

In another preferred embodiment of the present invention, the compositions and dosage forms of the invention are suitable for oral administration in solid or liquid form. The possible forms for oral administration are tablets, capsules, syrups, or solutions and can contain conventional excipients known in the pharmaceutical field, such as aggregating agents (for example, syrup, acacia, gelatin, sorbitol, tragacanth, or polyvinylpyrrolidone), fillers (for example, lactose, sugar, cornstarch, calcium phosphate, sorbitol, or glycine), disintegrants (for example, starch, polyvinylpyrrolidone, or microcrystalline cellulose) or a pharmaceutically acceptable surfactant such as sodium lauryl sulfate. Other dosage forms can be colloidal systems including, among others, nanoemulsions, nanocapsules, and polymeric nanoparticles.

The compositions for oral administration can be prepared by means of conventional methods of Galenic Pharmacy, such as mixing and dispersion. Tablets can be coated following methods known in the pharmaceutical industry.

The compositions and dosage forms can be adapted for parenteral administration as sterile solutions, suspensions, or lyophilisates of the products of the invention, using the suitable dose. Suitable excipients, such as pH buffering agents or surfactants, can be used.

The formulations mentioned above can be prepared using conventional methods, such as those described in the pharmacopeias of different countries and in other reference texts.

As it is used herein, the term "medicinal product" refers to any substance used for the prevention, diagnosis, relief, treatment, or cure of diseases in humans and animals.

The compounds, compositions, or dosage forms of the present invention can be administered by means of any suitable method, such as intravenous infusion and oral, topical, or parenteral routes. Oral administration is preferred for the convenience of the patients.

The administered amount of a compound of the present invention will depend on the relative efficacy of the chosen compound, the severity of the disease to be treated, and the weight of the patient. However, the compounds of this invention will be administered one or more times a day, for example 1, 2, 3, or 4 times a day, with a total dose of between 0.1 and 1000 mg/kg/day. It is important to take into account that it may be necessary to introduce variations in the dose, depending on the patient's age and condition, as well as modifications in the administration route.

The compounds and compositions of the present invention can be used together with other medicinal products in combined therapies. The other drugs can be part of the same composition or of another different composition for administration at the same time or at different times.

### COMBINED PREPARATION OF THE INVENTION

Therefore, another **aspect** of the invention relates to a combined preparation, hereinafter the combined preparation of the invention, which comprises:
a) a compound A which is selected from a compound of formula (I), and more preferably is pladienolide B, and
b) a compound B which is an antiangiogenic agent.

Preferably, the antiangiogenic agent is selected from: axitinib (Inlyta^{®}), bevacizumab (Avastin^{®}), cabozantinib (Cometriq^{®}), everolimus (Afinitor^{®}), lenalidomide (Revlimid^{®}), lenvatinib mesylate (Lenvima^{®}), pazopanib (Votrient^{®}), ramucirumab (Cyramza^{®}), regorafenib (Stivarga^{®}), sorafenib (Nexavar^{®}), sunitinib (Sutent^{®}), thalidomide (Synovir, Thalomid^{®}), vandetanib (Caprelsa^{®}), ziv-aflibercept (Zaltrap^{®}).

Even more preferably, the antiangiogenic agent is the compound sorafenib.

Another **aspect** relates to the use of the combined preparation of the invention for use thereof as a medicinal product, or alternatively for use thereof in therapy.

Another **aspect** relates to the combined preparation of the invention for separate, simultaneous, or sequential combined administration for the prevention, improvement, relief, or treatment of hepatocellular carcinoma in an individual.

As it is used herein, the term "active ingredient", "active substance", "pharmaceutically active substance", or "pharmaceutically active ingredient" means any component which potentially provides a pharmacological activity or another different effect on the diagnosis, cure, mitigation, treatment, or prevention of a disease, or which affects the bodily structure or function of humans or other animals. The term includes those components which promote a chemical change in the preparation of the drug and are present therein in a modified form intended to provide the specific activity or effect.

It should be highlighted that the term "combined preparation" or also referred to as "juxtaposition" herein means that the components of the combined preparation do not have to be present as a blend, for example in a true composition, to be available for the combined, separate, or sequential application thereof. The expression "juxtaposed" thereby implies that it is not necessarily a true combination, in view of the physical separation of the components.

The composition or combined preparation of the invention can be administered to a subject suffering from said pathologies by means of any of the following routes: intraperitoneal, intravenous, intramuscular, subcutaneous, intrathecal, intraventricular, oral, enteral, parenteral, intranasal, or dermal. In a preferred embodiment, the administration routes are preferably the intravenous route and the oral route.

Both the compositions of the present invention as well as the combined preparation or dosage forms of the invention, can be formulated for administration to an animal, and more preferably to a mammal, including humans, in a variety of forms known in the state of the art. In that sense, there can be, with limitation, in sterile aqueous solution or in biological fluids, such as serum. The aqueous solutions can be buffered or not buffered and have additional active or inactive components. The additional components include salts to modulate ionic strength, preservatives including, but not limited to, antimicrobial agents, antioxidants, chelating agents, and the like, and nutrients including glucose, dextrose, vitamins, and minerals. Alternatively, the compositions can be prepared for administration in solid form. The compositions can be combined with several inert vehicles or excipients, including but not limited to: binders such as microcrystalline cellulose, tragacanth gum, or gelatin; excipients such as starch or lactose; dispersing agents such as alginic acid or cornstarch; lubricants such as magnesium stearate, glidants such as colloidal silicon dioxide; sweetening agents such as sucrose or saccharin; or flavoring agents such as mint or methyl salicylate.

Such compositions or combined preparations and/or formulations thereof can be administered to an animal, including a mammal and, therefore, a human, in different ways, including, but not limited to, intraperitoneal, intravenous, intramuscular, subcutaneous, intrathecal, intraventricular, oral, enteral, parenteral, intranasal, or dermal.

The dosage for obtaining a therapeutically effective amount depends on a variety of factors, such as, for example, the age, weight, sex, tolerance, etc. of the mammal. In the sense used herein, the expression "therapeutically effective amount" refers to the amount comprising the active ingredient or ingredients of the invention which produce the desired effect, and it will generally be determined, among other factors, by the characteristics typical of said prodrugs, derivatives, or analogs and the therapeutic effect to be achieved. The "adjuvants" and "pharmaceutically acceptable vehicles" which can be used in said compositions are the vehicles known to those skilled in the art.

### SCREENING METHODS

Another aspect of the invention relates to a method of selecting therapeutic agents useful in the prevention, improvement, relief, and/or treatment of hepatocellular carcinoma which comprises:
a) contacting the compound to be analyzed with the SF3B1 polypeptide,
b) detecting the binding of said compound to be analyzed with the SF3B1 polypeptide.

More preferably, the hepatocellular carcinoma that is resistant to treatment with an antiangiogenic agent.

The compounds binding to the SF3B1 polypeptide would be identified as potential therapeutic agents for hepatocellular carcinoma, and more specifically for hepatocellular carcinoma that is resistant to treatment with an antiangiogenic agent. Even more preferably, the antiangiogenic agent is selected from: axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof. Still much more preferably, the antiangiogenic agent is sorafenib.

As mentioned, these assays can involve the complete SF3B1 polypeptide, a biologically active fragment thereof, or a fusion protein involving all or a portion of the SF3B1 polypeptide. The capacity of a compound to modulate SF3B1 activity can be determined, for example, by determining the capacity of SF3B1 to bind to or interact with a target molecule of said compound, directly or indirectly. They can also be activity assays, directly or indirectly measuring SF3B1 activity. It can be an expression assay, determining SF3B1 mRNA or SF3B1 protein expression directly or indirectly. These assays can also be combined with an *in vivo* assay, measuring the effect of a test compound on the symptoms of SF3B1-related diseases, and specifically hepatocellular carcinoma, preferably hepatocellular carcinoma that is resistant to treatment with an antiangiogenic agent (for example, but without limitation, on animal models or other model systems known in the art).

The compounds to be tested which are used in the method of selecting therapeutic agents are not limited to low weight molecular organic molecules, proteins (including antibodies), peptides, oligonucleotides, etc. They can be natural and/or synthetic compounds.

For example, antibodies capable of binding to an SF3B1 epitope, which can be used therapeutically, as discussed above, can also be used in immunohistochemical assays, such as Western blots, ELISAs, radioimmunoassays, immunoprecipitation assays, or other immunohistochemical assays known in the state of the art. The SF3B1 polypeptides can be used to immunize an animal in order to obtain polyclonal antibodies. Monoclonal antibodies can also be prepared by means of techniques which allow the production of antibodies by cultured cell lines including, among others, but without limitation, hybridomas, human B-cell hybridomas of cells. Techniques to produce chimeric, humanized, or synthetic antibodies are known.

The therapeutic agents identified by means of the selection method described herein can be used in an animal model or model of another type to determine the mechanism of action of said agent. Furthermore, the therapeutic agents selected by means of the method described herein would be used in the treatment of diseases occurring with SF3B18 alteration, and specifically hepatocellular carcinoma.

Another aspect of the invention describes a method of selecting therapeutic agents useful in the prevention, improvement, relief, and/or treatment of hepatocellular carcinoma, which comprises:
a) determining SF3B1 activity at an established concentration of the compound to be analyzed or in the absence of said compound,
b) determining SF3B1 activity at a concentration of the compound to be analyzed other than the concentration in a).

Compounds giving rise to a different SF3B1 activity would be identified as potential therapeutic agents for hepatocellular carcinoma, and preferably for hepatocellular carcinoma that is resistant to treatment with an antiangiogenic agent.

### METHODS OF OBTAINING DATA USEFUL FOR DIAGNOSIS

The diseases in which SF3B1 activity alteration may have diagnostic value, and specifically hepatocellular carcinoma, more specifically hepatocellular carcinoma that is resistant to treatment with an antiangiogenic agent, can be detected by measuring the amount of nucleic acids (DNA and/or RNA and/or mRNA) encoding SF3B1, or the amount of SF3B1 protein expressed, in comparison with normal cells. Oligonucleotides can be detected by means of methods well known in the state of the art (such as, for example, but without limitation, probes with labeled nucleotides, DNA-DNA or DNA-RNA hybridization, PCR amplification using labeled nucleotides, RT-PCR).

Methods for detecting SF3B1 protein expression also are well known in the state of the art, such as, for example, polyclonal or monoclonal antibodies, ELISA, radioimmunoassay (RIA), and FACS (fluorescence activated cell sorting).

Therefore, another aspect of the invention describes a method for collecting data useful in the diagnosis and/or prognosis of hepatocellular carcinoma, which comprises:
a) determining SF3B1 expression in a sample extracted from a mammal,
b) comparing the SF3B1 expression values obtained in a) with the standard values in healthy or sick mammals.

In a preferred embodiment, the hepatocellular carcinoma is resistant to treatment with an antiangiogenic agent. Even more preferably, the antiangiogenic agent is selected from: axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof.

Throughout the description and claims, the word "comprises" and its variants do not intend to exclude other technical features, additives, components, or steps. For those skilled in the art, other objects, advantages, and features of the invention will be inferred in part from the description and in part from putting the invention into practice. The following examples and drawings are provided by way of illustration and are not intended to be limiting of the present invention.

### EXAMPLES OF THE INVENTION

### Patients and samples

The study protocol was approved by the Ethics Committee of Hospital Universitario Reina Sofia in accordance with institutional and Good Clinical Practice guidelines (protocol number P117/02287) and in compliance with the Declaration of Helsinki. Informed consent was obtained from all patients or their relatives. Two independent retrospective cohorts of samples from HCC patients who underwent surgical resection or liver transplant were included: 1) Cohort-1: formalin-fixed paraffin-embedded (FFPE) samples including HCC and adjacent non-tumor tissue (n = 86); and, 2) Cohort-2: frozen samples comprising HCC tissue (n = 57), adjacent non-tumor tissue (n = 47), cirrhotic liver samples (n = 46), and normal liver samples from autopsies (n = 5). All these samples were obtained from the Andalusian Biobank (Cordoba Node), evaluated by means of liver histology and the diagnosis was confirmed by two independent and experienced pathologists. The patients' clinical data were obtained from electronic medical reports.

### Reagents

For *in vitro* and *in vivo* administrations, pladienolide-B (Santa Cruz, Heidelberg, Germany) was resuspended in DMSO (Sigma-Aldrich, Madrid, Spain). For *in vitro* administration, sorafenib (LC Laboratories, Woburn, USA) was dissolved in DMSO. In any case, the DMSO in the final solution did not exceed 0.2% (v/v). For *in vivo* tests, sorafenib was dissolved in Cremophor EL/ethanol (50:50) [12].

### Cell lines and treatments

HepG2, Hep3b, and SNU-387 (HB-8065) cell lines were purchased from ATCC (Manassas, USA) and cultured as recommended. The cells were kept at 37°C and 5% CO₂, and periodically validated by means of STR analysis (GenePrint, Promega, Barcelona, Spain) and analyzed to detect mycoplasma contamination [13-16]. THLE-2 cells were cultured according to the manufacturer's instructions [17]. In each experiment, cells were treated with pladienolide-B (10⁻⁷, 10⁻⁸, and 10⁻⁹M), sorafenib (5 µM) [18], or combination thereof. Positive controls [IGF-1 (10⁻⁶M)] and negative controls [Paclitaxel (10⁻⁷M)] were used.

### SF3B1 silencing by specific siRNA

A specific small interfering RNA (siRNA) for SF3B1 (s23851, Thermo Fisher, Madrid, Spain) and a commercial negative control (coding; Thermo Fisher) were used. For transfection, 120,000 SNU-387 cells and 150,000 Hep3b or HepG2 cells were seeded and transfected with 100 nM of SF3B1 siRNA (siSF3B1) using the Lipofectamine RNAiMAX reagent (Thermo Fisher).

### RNA isolation and reverse transcription

The total RNA from paraffin-embedded tissues was isolated using the Maxwell FFPE purification kit (Promega), the total RNA from frozen tissues was isolated using the AIIPrep DNA/RNA/Protein Kit (Qiagen, Madrid, Spain), and the total RNA from cell lines was isolated using TRI reagent (Sigma-Aldrich). RNA extraction was followed by treatment with DNase [13-15, 19]. The amount and purity of recovered RNA were determined using NanoDrop 2000 spectrophotometer (Thermo Fisher). The RNA (1 µg) was reverse transcribed using the RevertAid first-strand cDNA synthesis kit (Thermo Fisher).

### RNA expression analysis by means of a dynamic array of microfluidic-based qPCR and conventional qPCR

The expression levels of SF3B1 RNA, molecular markers, splice variants, and housekeeping genes were determined by microfluidic-based qPCR dynamic array [15, 20] in tissue samples and by conventional qPCR in cell lines and xenograft tumors. Primers specific for human transcripts (Complementary Table 1) were designed with the Primer3 software (Applied Biosystems, Foster City, CA). Pre-amplification, treatment with exonuclease, and dynamic array qPCR were developed using the Biomark system following manufacturer's instructions (Fluidigm, San Francisco, CA). Conventional qPCR was carried out using the Stratagene Mx3000p system with Brilliant III SYBR Green Master Mix (Stratagene, La Jolla, CA) [13, 14, 19]. In the case of tissue samples, the expression level of each transcript was adjusted by means of a normalization factor obtained from the expression levels of two housekeeping genes (ACTB and GAPDH) using Genorm 3.3 [21]. In the case of *in vitro* assays and preclinical *in vivo* model, the expression level of each transcript was adjusted by means of ACTB expression. In all cases, these housekeeping genes exhibited stable expression between experimental groups.

### In vitro assays

The determination of cell proliferation, migration, apoptosis, clone and tumorsphere formation was performed as published previously [13, 14, 19, 22].

### Xenograft model

Experiments with xenograft mice were conducted in accordance with the European Animal Care Regulations under the approval of the university/regional government research ethics committees. 5×10⁶ Hep3b cells (n = 16 mice; n = 32 tumors) in 50 µl of BME (Trevigen, Gaithersburg, MD) were subcutaneously grafted on both flanks of eight-week-old male Fox1nu/Foxn1nu nude mice (Janvier Labs, Le Genest-Saint-Isle, France). Tumor growth was monitored twice a week for 2 months by means of using a digital caliper. In the third week after grafting, when tumors were visible, the mice were treated with vehicle, pladienolide B, sorafenib, or the combination thereof (n = 4 mice/treatment; n = 8 tumors/treatment). Sorafenib (30 mg/kg) was administered dissolved in drinking water for 3 days [12]; whereas the water of the remaining mice was treated with cremophor/ethanol vehicle. Pladienolide-B (10-⁸M) was injected intratumorally on the second day of treatment with sorafenib [23]. After euthanizing the mice, each tumor was dissected for histopathological examination and for freezing or fixing. Tumor necrosis was evaluated after hematoxylin and eosin staining by expert pathologists.

### SF3B1 analysis by means of IHC

Immunohistochemistry (IHC) analysis was performed on a representative set of FFPE specimens having tumor regions and adjacent non-tumor regions from patients diagnosed with HCC (n = 16). The SF3B1 monoclonal antibody (ab172634, Abcam, Cambridge, United Kingdom) was used at a 1:250 dilution [11]. Two independent pathologists performed histopathological analyses of the tumors following a blinded protocol. In the analysis, +, ++, +++ indicate low, moderate, and high staining intensity.

### In silico analysis of SF3B1 expression in HCC cohorts

GEPIA2 was used to analyze SF3B1 expression level and survival curves in TCGA. The Oncomine database was used to analyze SF3B1 expression levels in other cohorts *in silica.* Wurmbach liver (10 normal liver vs. 35 HCC) [24], Mas liver (19 normal liver vs. 38 HCC) [25], Roessler liver (21 normal liver vs. 22 HCC), and Roessler liver 2 (220 normal liver vs. 225 HCC) [26].

### Statistical analysis

Data is expressed as mean ± standard error of the mean (SEM), as log2 change, or as relative levels in comparison with corresponding controls (established at 100%). Data was evaluated to determine the heterogeneity of variance by means of the Kolmogorov-Smirnov test, and parametric (Student's t-test) or non-parametric (Mann-Whitney U) tests were performed accordingly. Spearman or Pearson bivariate correlations were performed for quantitative variables according to normality. The significant relationship between categorized mRNA expression and patient survival was studied using the long-rank p-value method. P-values of less than 0.05 were considered statistically significant. All statistical analyses were performed using the GraphPad Prism 6.0 software (La Jolla, CA, USA).

### Results

SF3B1 is overexpressed in HCC, correlates with oncogenic splice variants, and is associated with overall survival.

SF3B1 was significantly overexpressed in two independent cohorts of HCC samples (Table 1) in comparison with adjacent non-tumor tissues and cirrhotic samples (Figure 1A). These results were additionally corroborated *in silico* in 4 cohorts of HCC samples (complementary Figure 1). SF3B1 was also overexpressed in HCC samples in comparison with normal liver in the TGCA dataset (Figure 1B). Furthermore, SF3B1 expression was greater in the three liver cell lines used (HepG2, Hep3b, and SNU-387) in comparison with THLE-2 cells, which have been derived from normal hepatocytes (Complementary Figure 2). SF3B1 expression in HCC samples from cohort-1 and cohort-2 directly correlated with the expression of three oncogenic splice variants, KLF6-SV1, CCDC50S, and BCL-XL (Figure 1C; R> 0.370; p <0.01), the expression of which was elevated in HCC samples (Complementary Figure 3). Consistently, SF3B1 protein levels evaluated by means of IHQ were significantly higher in HCC in comparison with the adjacent non-tumor regions (Figure 1D).

It should be pointed out that SF3B1 expression levels correlated with tumor diameter, node number, degree of tumor differentiation, and microvascular invasion, but not with the etiology of the underlying liver disease in Cohort-2 (Figure 2A). This information was not fully disponible for Cohort-1 and only correlation between SF3B1 and node number was found (Complementary Figure 4). Surprisingly, SF3B1 was associated with overall survival (Figure 2B). Specifically, higher SF3B1 expression levels correlated with lower overall survival rates in TCGA (HR = 1.6, p = 0.006) and also tended to correlate in Cohort-1 (HR = 1.99, p = 0.063), whereas this effect was less pronounced in Cohort 2 (HR = 3.4, p = 0.134), which is made up of less aggressive HCC with better prognosis (Table 1).

SF3B1 silencing reduced the aggressivity of HCC cell lines.

To analyze the involvement of SF3B1 in HCC physiopathology, a specific siRNA (siSF3B1) was used to significantly reduce SF3B1 expression levels [mRNA (Figure 3A, confirmed in HepG2, Hep3b, and SNU-387) and protein levels (Figure 3B, confirmed in Hep3b)] in comparison with cells transfected with the control. SF3B1 silencing significantly reduced cell proliferation in a time dependent manner (Figure 3C), as well as migration (Figures 3D-E) in the three cell lines.

### Pharmacological blocking of SF3B1 with pladienolide-B reduced aggressivity of HCC cell lines

Pladienolide-B, a pharmacological SF3B1 inhibitor, exhibited a strong dose-dependent inhibitory effect on cell proliferation in the three HCC cell lines, whereas it only exerted a moderate effect on the cell line derived from normal hepatocytes (THLE-2) (Figure 4A and complementary Figure 5). In particular, the three HCC cell lines exhibited a dose- and time-dependent response similar to pladienolide-B, since the dose of 10⁻⁹ M did not have a significant effect on cell proliferation, whereas 10⁻⁸ M and 10⁻⁷ M of pladienolide-B clearly inhibited cell proliferation, particularly at 48-72 h (Figure 4A). In the case of THLE-2 cells, only a dose of 10⁻⁷M reduced cell viability, whereas doses of 10⁻⁹M and 10⁻⁸M did not exert any significant inhibition (Figure 4A). For these reasons, the dose of 10⁻⁸M was selected for subsequent experiments.

Wound healing assays demonstrated that treatment with pladienolide-B significantly reduced the migration capacity of the three HCC cell lines (Figures 4B and C). Furthermore, the formation of tumorspheres was significantly reduced in response to pladienolide-B. In fact, the mean tumorsphere size was reduced in the three HCC cell lines 10 days after treatment with pladienolide-B (Figures 4D and E). Furthermore, clonogenic assays demonstrated that the number of colonies formed was significantly smaller in cells treated with pladienolide-B in comparison with cells treated with vehicle (Figures 4F and G). Lastly, DAPI staining revealed that pladienolide-B significantly increased apoptosis in HCC cells (Figures 4H and I).

Pladienolide-B improved the effects of sorafenib on more aggressive HCC cell lines.

In the experiments combining pladienolide-B (10⁻⁸ M) and sorafenib (5 nM), pladienolide-B, as expected, did not reduce the viability of THLE-2 cells after 72 h, whereas sorafenib significantly reduced same (Figure 4J). In contrast, pladienolide-B exhibited an effect comparable to that exerted by sorafenib in terms of proliferation in the three HCC cell lines (Figure 4J). It should be pointed out that the combination of sorafenib and pladienolide-B exerted significantly more pronounced effects than sorafenib alone on more aggressive Hep3b and SNU-387 cell lines (Figure 4J).

Pladienolide-B reduced the *in vivo* growth of HCC cells xenografted on nude mice.

The effect of pladienolide-B on tumor growth *in vivo* was evaluated in xenografts induced by Hep3b (Figure 5A). A single intratumoral dose of pladienolide-B significantly reduced the growth of said tumors in comparison with tumors treated with vehicle (Figure 5B). The effect of pladienolide-B was comparable to the effect exerted by orally administered sorafenib.

In terms of histopathology, tumors treated with sorafenib exhibited greater necrosis than tumors treated with vehicle (Figure 5C).

SF3B1 silencing or blocking reduced the expression of aggressiveness markers and oncogenic splice variants.

The molecular mechanisms associated with SF3B1 silencing and blocking in the Hep3b cell line was examined. SF3B1 silencing and/or treatment with pladienolide-B (10⁻⁸ M) in cell cultures and xenografted tumors reduced the expression of different tumor markers such as CDK4, CDK6, or CD24 (Figure 6A). Furthermore, SF3B1 silencing and/or pharmacological blocking induced a selective reduction of key oncogenic splice variants which correlated with SF3B1 expression in human HCC samples. In fact, *in vitro* SF3B1 silencing or blocking reduced KLF6-SV1 and BCL-XL expression (Figure 6B), whereas *in vivo* treatment with pladienolide-B reduced KLF6-SV1 expression in tumors derived from Hep3b (Figure 6B)

### CLAUSES

1. An SF3B1 modulating agent, for the prevention, improvement, relief, and/or treatment of hepatocellular carcinoma in an individual.
2. The SF3B1 modulating agent for use thereof according to the preceding claim, wherein the modulating agent is selected from a list comprising:
   a) an organic molecule,
   b) an RNA molecule,
   c) an antisense oligonucleotide,
   d) an antibody, or e) a ribozyme.
3. The modulating agent for use thereof according to any of claims 1-2, which is an organic molecule of formula (I) 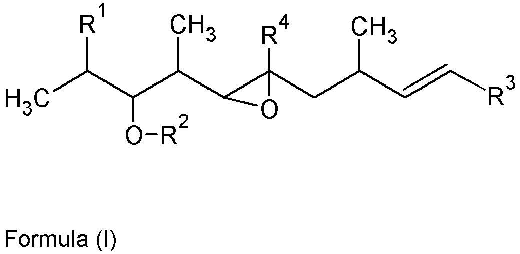 wherein
   R¹ is H, OH, or alkoxy;
   R² is H or CH3;
   R⁴ is H or CH3;
   R³ is alkyl, cycloalkyl, aryl, or heterocycloalkyl;
   or a pharmaceutically acceptable salt thereof;
4. The compound for use thereof according to claim 3, wherein the compound of formula (I) is pladienolide B of formula (II): 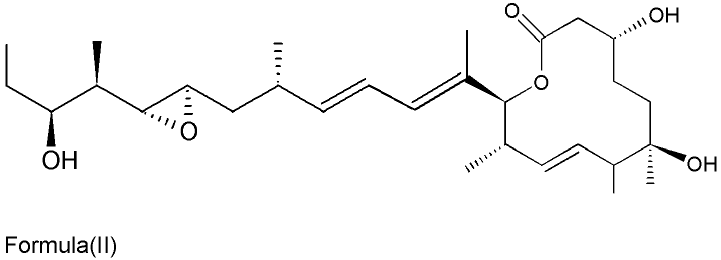
5. The compound for use thereof according to any of claims 3-4, wherein the individual has hepatocellular carcinoma that is resistant to treatment with an antiangiogenic agent.
6. The compound for use thereof according to the preceding claim, wherein the antiangiogenic agent is selected from: axitinib, bevacizumab, cabozantinib, everolimus, lenalidomide, lenvatinib mesylate, pazopanib, ramucirumab, regorafenib, sorafenib, sunitinib, thalidomide, vandetanib, ziv-aflibercept, or any of the combinations thereof.
7. The compound for use thereof according to any of claims 3-6, wherein the antiangiogenic agent is sofarenib.
8. A composition comprising the compound for use thereof according to any of claims 3-7, which is a pharmaceutical composition.
9. The composition according to claim 8, further comprising a pharmaceutically acceptable vehicle.
10. The composition according to any of claims 8-9, further comprising another active ingredient.
11. A combined preparation comprising:
   a) a compound according to any of claims 3-7, and
   b) an antiangiogenic agent.
12. The combined preparation according to the preceding claim for the prevention, improvement, relief, or treatment of hepatocellular carcinoma in an individual.
13. The combined preparation for use thereof according to the preceding claim, wherein the hepatocellular carcinoma that is resistant to treatment with an antiangiogenic agent.
14. The combined preparation for use thereof according to any of claims 12-13, wherein the antiangiogenic agent is selected from: axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof.
15. A method for collecting data useful in the diagnosis and/or prognosis of hepatocellular carcinoma, which comprises:
   a) determining SF3B1 expression in a sample extracted from a mammal,
   b) comparing the SF3B1 expression values obtained in a) with the standard values in healthy or sick mammals.
16. A method of selecting therapeutic agents useful in the prevention, improvement, relief, and/or treatment of hepatocellular carcinoma, which comprises:
   a) determining SF3B1 activity at an established concentration of the compound to be analyzed or in the absence of said compound,
   b) determining SF3B1 activity at a concentration of the compound to be analyzed other than the concentration in a).
17. A method for collecting data useful in the diagnosis and/or prognosis of hepatocellular carcinoma, which comprises:
   a) determining SF3B1 expression in a sample extracted from a mammal,
   b) comparing the SF3B1 expression values obtained in a) with the standard values in healthy or sick mammals.
18. The method according to any of claims 15-17, wherein the hepatocellular carcinoma that is resistant to treatment with an antiangiogenic agent.
19. The method according to the preceding claim, wherein the antiangiogenic agent is selected from: axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof.
20. The method according to any of claims 15-19, wherein the antiangiogenic agent is sorafenib.

## Claims

1. A compound of formula (I) 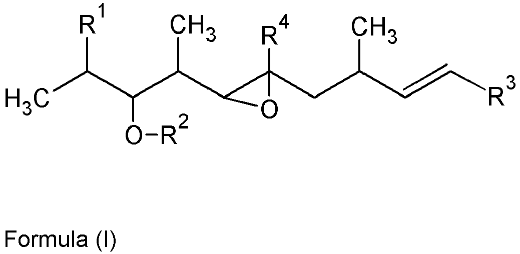 wherein
R¹ is H, OH, or alkoxy;
R² is H or CH3;
R⁴ is H or CH3;
R³ is alkyl, cycloalkyl, aryl, or heterocycloalkyl;
or a pharmaceutically acceptable salt thereof;
for the prevention, improvement, relief, or treatment of hepatocellular carcinoma in an individual with hepatocellular carcinoma in combination with an antiangiogenic agent.

2. The compound for use thereof according to claim 1, wherein the antiangiogenic agent is selected from the list consisting of axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof.

3. The compound for use thereof according to any of claims 1-2, wherein the antiangiogenic agent is selected from the list consisting of sorafenib, sunitinib, everolimus, erlotinib, or any of the combinations thereof.

4. The compound for use thereof according to any of claims 1-3, wherein the antiangiogenic agent is sorafenib.

5. The compound for use thereof according to any of claims 1-4, wherein the compound of formula (I) is pladienolide B of formula (II):

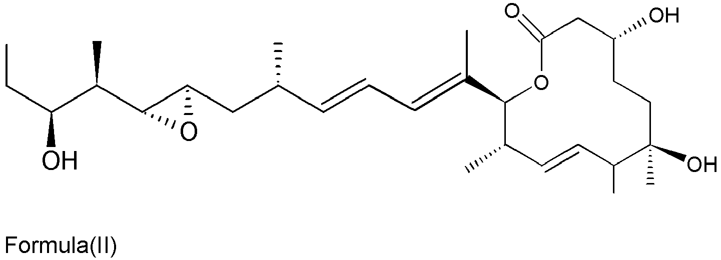

6. The compound for use thereof according to any of claims 1-5, wherein the individual has hepatocellular carcinoma that is resistant to treatment with antiangiogenic agents.

7. A composition comprising the compound for use thereof according to any of claims 1-6, which is a pharmaceutical composition.

8. The composition according to claim 7, further comprising a pharmaceutically acceptable vehicle.

9. The composition according to any of claims 7-8, further comprising another active ingredient.

10. A combined preparation comprising:
a) a compound as described in any of claims 1-6, and
b) an antiangiogenic agent which is selected from the list consisting of: sorafenib, sunitinib, everolimus, erlotinib, or any of the combinations thereof.
for the prevention, improvement, relief, or treatment of hepatocellular carcinoma in an individual with hepatocellular carcinoma that is resistant to treatment with an antiangiogenic agent which is selected from the list consisting of: sorafenib, sunitinib, everolimus, erlotinib, or any of the combinations thereof.

11. The combined preparation according to claim 10, wherein the antiangiogenic agent is selected from the list consisting of axitinib, bevacizumab, cabozantinib, everolimus, lenalidomide, lenvatinib mesylate, pazopanib, ramucirumab, regorafenib, sorafenib, sunitinib, thalidomide, vandetanib, ziv-afliberceptm, or any of the combinations thereof

12. The combined preparation according to any of claims 10-11, wherein the antiangiogenic agent is selected from the list consisting of sorafenib, sunitinib, everolimus, erlotinib, or any of the combinations thereof.

13. The combined preparation according to any of claims 10-12, wherein the antiangiogenic agent is sorafenib.

14. The combined preparation according to any of claims 10-13, for the prevention, improvement, relief, or treatment of hepatocellular carcinoma.

15. The combined preparation according to any of claims 10-13, for the prevention, improvement, relief, or treatment of hepatocellular carcinoma in an individual with hepatocellular carcinoma that is resistant to treatment with antiangiogenic agents.

16. A method for collecting data useful in the diagnosis and/or prognosis of hepatocellular carcinoma that is resistant to treatment with sorafenib, which comprises:
a) determining SF3B1 expression in a sample extracted from a mammal,
b) comparing the SF3B1 expression values obtained in a) with the standard values in healthy or sick mammals.

17. A method of selecting therapeutic agents useful in the prevention, improvement, relief, and/or treatment of hepatocellular carcinoma in an individual who is resistant to treatment with sorafenib, which comprises:
a) determining SF3B1 activity at an established concentration of the compound to be analyzed or in the absence of said compound,
b) determining SF3B1 activity at a concentration of the compound to be analyzed other than the concentration in a).

18. A method for collecting data useful in the diagnosis and/or prognosis of hepatocellular carcinoma in an individual who is resistant to treatment with sorafenib, which comprises:
a) determining SF3B1 expression in a sample extracted from a mammal,
b) comparing the SF3B1 expression values obtained in a) with the standard values in healthy or sick mammals.
